# EUROPEAN PATENT APPLICATION

(11) **EP 2 241 309 A2**
(43) Date of publication of application: **20.10.2010**
(21) Application number: 10168775.4
(22) Date of filing: 10.07.2002
(51) Int. Cl.: A61K 9/16, A61K 39/39, A61K 39/04

(54) **Methods for encapsulation of proteins and adjuants in microspheres**

(30) Priority: 10.07.2001 US 304590 P; 09.11.2001 US 346013 P
(62) Divisional of application: 02752243.2
(71) Applicant: CORIXA CORPORATION, Seattle, WA 98101 (US)
(72) Inventor: Johnson, Mark E., Bellevue, WA 98006 (US); Evans, Jay T., Hamilton, MT 59840 (US); Kern, Jeffrey A., Hamilton, MT 59840 (US)
(74) Representative: Kershaw, Alison Lesley

(57) **Abstract**

A method for encapsulating a protein into microspheres comprising:
(a) solubilizing the protein in the presence of a hydrophobic ion pairing (HTP) agent and an organic solvent to produce an organic phase comprising the protein;
(b) dissolving a polymer in the organic solvent or in the organic phase; and
(c) preparing microspheres from a polymer solution, wherein the polymer solution comprises the organic phase, the protein, and the polymer.

## Description

This application claims the benefit of United States provisional patent application serial numbers 60/304,590, filed July 10, 2001, and 60/346,013, filed November 9, 2001, the entire contents of each of which are incorporated herein by reference. Throughout this application various publications are referenced. The disclosures of these publications in their entireties are hereby incorporated by reference into this application in order to describe more fully the state of the art to which this invention pertains.

### TECHNICAL FIELD OF THE INVENTION

The invention relates to formulations, compositions and methods that can be used for the delivery of vaccines and adjuvants. More particularly, the invention relates to microspheres and methods for preparing microspheres that enable more efficient and effective delivery of protein vaccines and adjuvants.

### BACKGROUND OF THE INVENTION

New vaccines are in development for the prevention, as well as the treatment, of cancers and chronic infectious diseases. The most effective vaccines will likely elicit CTL responses in addition to T-helper responses and antibodies. An attractive mode of vaccine delivery is via encapsulation in microspheres. Due to the insolubility of most proteins in organic media, however, microspheres encapsulating proteins typically need to be made by a double-emulsion method. Microsphere formulations made by double-emulsion methods often have undesirable release kinetics (e.g., high initial burst and/or very slow additional release over time), as indicated by *in vitro* release studies.

There remains a need for more efficient and effective means of delivery of protein vaccines, particularly methods that provide desirable release kinetics while also maintaining protein stability.

The invention provides a composition comprising an adjuvant encapsulated in biodegradable polymeric microspheres and a pharmaceutically acceptable carrier. In one embodiment, a protein is co-encapsulated with the adjuvant in the microspheres. Typically, the protein comprises an antigen associated with cancer, autoimmune disease or infectious disease. In accordance with the invention, proteins and adjuvants can be delivered via encapsulation in polymeric microspheres either via co-encapsulation in the same microspheres, or co-administered as encapsulated protein in a first set of microspheres and encapsulated adjuvant in a second set of microspheres. In preferred embodiments, proteins and/or adjuvants are encapsulated into microspheres via hydrophobic ion pairing (HIP).

The invention further provides a method for encapsulating a protein into microspheres wherein HIP is applied to solubilize proteins in an organic medium. The method comprises solubilizing the protein in the presence of a HIP agent and an organic solvent to produce an organic phase comprising the protein. The method further comprises dissolving a polymer in the organic solvent or in the organic phase. Microspheres are then prepared from a polymer solution, wherein the polymer solution comprises the organic phase, the protein, and the polymer. In a preferred embodiment, the protein is extracted from an aqueous solution into the organic phase. In another embodiment, the solubilizing comprises combining the organic solvent with a dried HIP agent-protein complex. The HIP agent-protein complex can be dried by lyophilization or evaporation, for example.

Because hydrophobic ion pairing allows extraction of protein into an organic medium, the method enables preparation of microsphere formulations with a single emulsion. The resulting microspheres display desirable release kinetics, i.e., low initial burst and controlled release of the protein over time. Surprisingly, the method of encapsulation has been demonstrated to encapsulate proteins of larger sizes than expected, and these encapsulated proteins have demonstrated effective release under physiological conditions. Such encapsulated proteins elicit strong and comprehensive immune responses, including both cellular and humoral immune responses. Examples of proteins to be encapsulated in microspheres of the invention include proteins having a molecular weight of at least about 3 kDa, preferably at least about 8 kDa, more preferably at least about 20 kDa. Larger proteins can also be encapsulated into microspheres in accordance with the invention, including those having a molecular weight of at least about 50 kDa, including ICD of her-2/neu, which has a molecular weight of about 66 kDa. Protein antigens to be encapsulated into microspheres of the invention can also be of considerable length, including antigens of at least about 20 amino acid residues in length. Preferably, the protein antigen has a length of at least about 60 amino acid residues, more preferably, at least about 80 amino acids, and most preferably, at least about 100 amino acids in length.

In one embodiment, the HIP agent is an anionic HIP agent, such as docusate sodium, and the HIP agent is present in stoichiometric amounts equal to or greater than the number of net positive charges on the protein. In another embodiment, the HIP agent is a cationic HIP agent, such as dimethyldioctadecyl-ammonium bromide (DDAB18); 1,2-dioleoyloxy-3-(trimethylammonium)propane (DOTAP); or cetrimonium bromide (CTAB). In this embodiment, the HIP agent is present in stoichiometric amounts equal to or greater than the number of net negative charges on the protein. Preferably, the organic medium has a ratio of HIP agent to protein of up to about 70:1.

The HIP agent and the aqueous solution are selected in accordance with the characteristics of the protein to be encapsulated. Typically, for proteins having an isoelectric point (pI) at or below 7.0, an anionic HIP agent is preferred. Likewise, for proteins having a pI greater than or equal to 7.0, a cationic HIP agent is preferred. For encapsulation of a protein having a pI of about 7.0, either a cationic or anionic HIP agent can be used. The pH of the aqueous solution can be adjusted to achieve the appropriate charge characteristics. Typically, aqueous solutions having low salt concentrations are preferred. In one embodiment, the aqueous solution has a total salt concentration of less than about 30 mM. The method is suitable for proteins having a variety of isoelectric points, including those having a pI of at least about 7.5, at least about 8.0, up to about 6.0, up to about 6.5, as well as pI's of up to or greater than about 7.0.

Examples of organic solvents suitable for use with the method of the invention include, but are not limited to, methylene chloride, dichloromethane, chloroform, ethylacetate, or dimethylsulfoxide. The microspheres can be prepared by a variety of methods known in the art, including a single oil-in-water emulsion, a double oil-in-water emulsion, spray drying or coacervation of the polymer solution. An advantage of the method of the invention, is that it allows for preparation of the microspheres by a single emulsion, and thereby obtaining microspheres exhibiting improved release kinetics. Preferably, at least about 90% of the microspheres are about 1 to about 10 µm in diameter.

A preferred polymer for use in the method comprises poly(lactide-co-glycolide) (PLG). Other suitable polymers include poly(lactide), poly(caprolactone), poly(hydroxybutyrate) and/or copolymers thereof. Preferably, the polymer solution further comprises an adjuvant, such as MPL, saponin, aluminum phosphate, calcium phosphate, aminoalkylglucosaminide phosphate, isotucerasol, cell wall skeleton, and/or a CpG-containing oligonucleotide.

The protein to be encapsulated in the microspheres typically comprises an antigen, such as an antigen associated with cancer, autoimmune disease or an infectious disease. In one embodiment, the infectious disease is tuberculosis. Representative tuberculosis antigens include Mtb8.4, TbH9 (Mtb 39A), 38-1, Mtb41, Mtb40, Mtb32A, Mtb9.9A, Mtb9.8, Mtb16, Mtb72f, Mtb59f, Mtb88f, Mtb71f, Mtb46f and Mtb31f. In another embodiment, the antigen is associated with breast cancer, such as the intracellular domain (ICD) or extracellular domain (ECD-PD) of her-2/neu.

The invention further provides a composition comprising a protein encapsulated in microspheres produced by the method of the invention. The composition preferably further comprises an adjuvant, such as MPL, saponin, AS-2, aluminum phosphate, calcium phosphate, aminoalkylglucosaminide phosphate, isotucerasol, cell wall skeleton, and/or a CpG-containing oligonucleotide. In one embodiment, the adjuvant is co-encapsulated with the antigen in the microspheres. In other embodiments, the adjuvant is co-administered with the encapsulated protein antigen. Also provided are a composition comprising one or more adjuvants encapsulated in microspheres, and methods of delivering adjuvants provided in such a composition.

The invention also provides methods for delivering an antigen to a subject, for eliciting an immune response to an antigen in a subject, and for treating or preventing cancer, autoimmune disease or infectious disease in a subject. These methods comprise administering to the subject a composition of the invention. Typically, the immune response elicited by the method of the invention includes both a humoral and a cellular immune response.

### BRIEF DESCRIPTION OF THE FIGURES

Figure 1 is a graph showing DPV release plotted as a function of time, in days, for five microsphere formulations. The formulations included JA-024, 40% ethyl myristate (C14) (diamonds); AS-011 (squares); AS-012, 15% cholesterol (triangles); AS-014, 20% ethyl caprate (C10) (X's); AS-013, 20% ethyl stearate (C18) (asterisks); and JA-002, RG-502 (+'s).

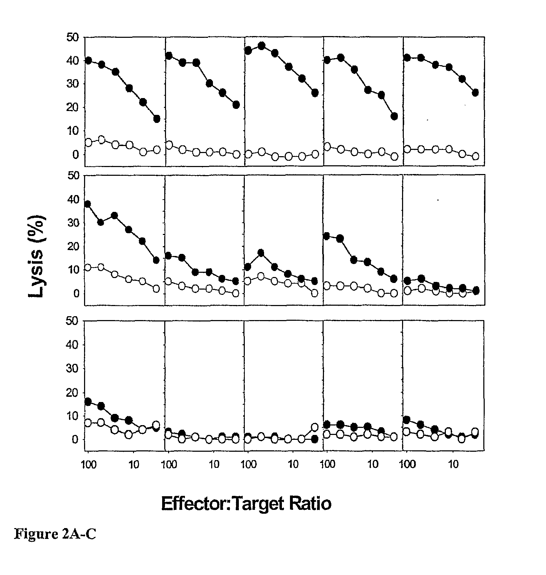

Figures 2A-C are graphs depicting the results of a CTL assay from an experiment in which mice, in groups of five, were given two 5 µg immunizations of protein subcutaneously three weeks apart. Mtb8.4 protein-microspheres prepared using the HIP technique of the invention were administered to one group (Fig. 2A); Mtb8.4 protein plus MPL/saponin adjuvant combination was administered to another group (Fig. 2B); and Mtb8.4 protein alone was administered to a third group (Fig. 2C). Closed circles represent lysis of targets transduced with Mtb8.4 DNA. Open circles represents lysis of control EL4 targets.
Figures 3A-C are graphs showing that Mtb8.4 protein microspheres prepared using the HIP technique of the invention elicited antibody responses (IgG1; Fig. 3A) that were as strong as those elicited by the MPL/saponin adjuvant combination (Fig. 3B) and significantly stronger than those elicited by protein alone (Fig. 3C). The results from individual mice are shown as individual lines.
Figure 4 shows CTL responses measured for groups of mice from pooled spleens (n=7) using a chromium release assay after one *in vitro* stimulation of mouse splenocytes. Closed symbols represent lysis of targets transduced with Mtb8.4 DNA. Open symbols represent lysis of control EL4 targets. Strong CTL responses were measured in the group receiving protein-microspheres (circles). This response was significantly stronger than the response measured for the protein plus MPL/saponin group (triangles) and, remarkably, comparable to the response for the group that received DNA (squares).
Figure 5 shows IFNγ release from rMtb8.4 activated spleen cell cultures as determined by intracellular cytokine (ICC) assay. The percentage of CD8+IFNγ+ cells in the spleens of individual mice was measured 14 days following a single SC or ID immunization. MJ-071b indicates r Mtb8.4 encapsulated microspheres without MPL.
Figure 6 shows IFNγ release from spleen cell cultures as determined by enzyme linked immunosorbant assay (ELISA). Spleen cell cultures (pooled spleens from 3 mice) were activated with recombinant Mtb8.4 for 72 hours. MJ-071b indicates Mtb8.4 encapsulated microspheres without MPL.
Figure 7 shows percent specific lysis by CTL response of Mtb8.4-EL4 targets by CD8+ T cells following 5 day activation with irradiated Mtb8.4-EL4 cells. MJ-071b indicates r Mtb8.4 encapsulated microspheres without MPL.

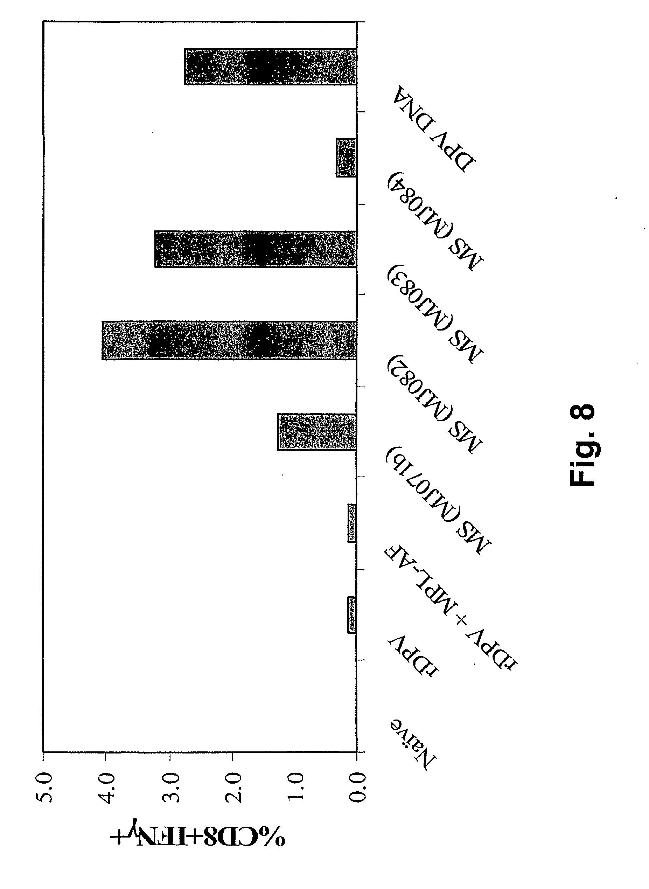

Figure 8 shows the percentage of Mtb8.4 cells reactive to CD8+ cells by ICC for IFNγ at 14 days following primary immunization. MJ-073 indicates Mtb8.4 encapsulated microspheres without MPL; MJ-082 indicates microspheres containing both Mtb8.4 and adjuvant (MPL) where MPL was added to the organic phase after protein extraction and before addition to the process media; MJ-083 indicates microspheres containing both Mtb8.4 and adjuvant (MPL) where MPL was incorporated via an inner aqueous phase; and MJ-084 indicates microspheres containing both Mtb8.4 and adjuvant (MPL) where MPL was added to the process media in place of the polyvinyl alcohol (PVA).
Figure 9 shows the percentage of Mtb8.4 cells reactive to CD4+ cells by ICC for IFNγ at 14 days following primary immunization.
Figure 10 shows IgG1 antibody levels in serum from C57BL/6 mice 14 days following secondary immunization with Mtb8.4-microspheres.
Figure 11 shows IgG2b antibody levels in serum from C57BL/6 mice 14 days following secondary immunization with Mtb8.4 microspheres.
Figure 12 shows Mtb8.4 dose dependent percent specific lysis by CTL response of ) Mtb8.4-EL4 targets by CD8+ T cells following 5 day activation with irradiated Mtb8.4-EL4 cells.
Figure 13 shows Mtb8.4 dose dependent percentage of Mtb8.4 cells reactive to CD4+ cells by ICC for IFNγ at 14 days following primary immunization.
Figure 14 shows Mtb8.4 dose dependent percentage of Mtb8.4 cells reactive to CD8+ cells by ICC for IFNγ at 14 days following primary immunization.
Figures 15A-D are bar graphs illustrating T-cell responses measured by CD8 (15A and 15C) and CD4 (15B and 15D) ICCS with T-cells harvested two-weeks after a single immunization.
Figures 16A-B are bar graphs showing IFN-γ release from spleen cells harvested two-weeks after the primary immunization. Spleens were pooled for this assay.
Figures 17A-D are bar graphs showing IgG1 and IgG2b serum antibody responses specific for Mtb8.4 in sera obtained two-weeks after the second immunization.
Figure 18 is a bar graph showing CTL responses measured two weeks after a single immunization.
Figure 19 is a bar graph showing IFN-gamma release from spleen cell cultures measured from mice receiving either a primary immunization or a primary and a secondary immunizations.
Figure 20 is a graph illustrating IgG2b serum antibody specific for Mtb8.4 measured two weeks after the mice received a second immunization.
Figure 21 is a graph illustrating IgG1 serum antibody specific for Mtb8.4 measured two weeks after the mice received a second immunization.
Figure 22 is a graph showing tumor growth in naive mice (n=8) over time.
Figures 23 and 24 are graphs showing tumor growth over time in the positive control groups of mice (n=8). Mice were immunized twice with 25 µg of ICD protein formulated in Montanide adjuvant or twice with ICD-DNA.
Figures 25-28 are graphs showing tumor growth over time in the groups of mice (n=8) that received ICD protein microspheres. Mice were immunized twice with 25 µg of ICD protein encapsulated in microspheres, lot number AM049, AM050, AM051, or AM052, respectively.

### DETAILED DESCRIPTION OF THE INVENTION

The invention disclosed herein is based on the surprising discovery that a strong and comprehensive immune response can be obtained to vaccine compositions comprising protein and adjuvant co-encapsulated in microspheres. Strong immune responses have been obtained using antigens associated with cancer, autoimmune disease and infectious disease. Co-administration of encapsulated protein antigens and separately encapsulated adjuvant elicits a comprehensive immune response, and an even stronger comprehensive immune response can be obtained using protein and adjuvant co-encapsulated in the same set of microspheres. Thus, despite the hydrophobicity of adjuvants tested in the examples provided herein, effective adjuvant delivery has been achieved using adjuvant encapsulated in microspheres. While encapsulation of protein and/or adjuvant can be achieved by a variety of methods known in the art, the encapsulation is preferably performed via hydrophobic ion pairing as described herein.

Surprisingly, the method of encapsulation has been demonstrated to encapsulate proteins of substantial size, and these encapsulated proteins have demonstrated effective release under physiological conditions. Such encapsulated proteins elicit strong and comprehensive immune responses, including both cellular and humoral immune responses. Examples of proteins to be encapsulated in microspheres of the invention include proteins having a molecular weight of at least about 3 kDa, preferably at least about 8 kDa, more preferably at least about 20 kDa. Larger proteins can also be encapsulated into microspheres in accordance with the invention, including those having a molecular weight of at least about 50 kDa, including ICD of her-2/neu, which has a molecular weight of about 66 kDa. Protein antigens to be encapsulated into microspheres of the invention can also be of considerable length, including antigens of at least about 20 amino acid residues in length. Preferably, the protein antigen has a length of at least about 60 amino acid residues, more preferably, at least about 80 amino acids, and most preferably, at least about 100 amino acids in length.

Hydrophobic ion pairing (HIP) involves stoichiometric replacement of polar counter ions with a species of similar charge but less easily solvated. As disclosed herein, the invention provides a method that uses HIP to change the solubility properties of proteins, allowing extraction of the protein into an organic solvent, such as methylene chloride. Docusate sodium (Bis(2-ethylhexyl) sodium sulfosuccinate) is one example of a suitable ion-pairing agent. In one embodiment, methylene chloride containing docusate sodium is mixed with an aqueous protein solution. This results in ion-pairing of the docusate ion with the protein and subsequent partitioning of the protein into the oil phase. Dissolution of the protein in methylene chloride allows the protein to be encapsulated in microspheres prepared via a single oil-in-water emulsion method.

Microspheres prepared by this method exhibit desirable protein release characteristics, including low initial burst release and a gradual release of protein over time. The release kinetics may be further modified by incorporating additives such as cholesterol and esters of fatty acids, which are soluble in the organic solvent. The invention provides microsphere formulations encapsulating a protein antigen, wherein the protein antigen is released gradually over time. Use of HIP to produce microspheres in accordance with the invention allows for a more even distribution of the protein within the microspheres, and reduces aggregation of the protein in the microspheres.

### Definitions

All scientific and technical terms used in this application have meanings commonly used in the art unless otherwise specified. As used in this application, the following words or phrases have the meanings specified.

As used herein, "protein" or "polypeptide" means a polymer of at least about 20 or, more typically, at least about 50 amino acids. Such proteins or polypeptides have primary, secondary, tertiary and, in some cases, quaternary, structures. The protein or polypeptide can be isolated from natural sources, produced by recombinant techniques or chemically synthesized.

As used herein, "immune response" includes the production of antibodies, production of immunomodulators such as IFN-γ, and induction of CTL activity. The elicitation of an immune response includes the initiation, stimulation or enhancement of an immune response. A "comprehensive immune response" refers to a response that includes both humoral and cellular immune responses.

As used herein, to "prevent" or "protect against" a condition or disease means to hinder, reduce or delay the onset or progression of the condition or disease.

As used herein, "antigen-presenting cell" or "APC" means a cell capable of handling and presenting antigen to a lymphocyte. Examples of APCs include, but are not limited to, macrophages, Langerhans-dendritic cells, follicular dendritic cells, B cells, monocytes, fibroblasts and fibrocytes. Dendritic cells are a preferred type of antigen presenting cell. Dendritic cells are found in many non-lymphoid tissues but can migrate via the afferent lymph or the blood stream to the T-dependent areas of lymphoid organs. In non-lymphoid organs, dendritic cells include Langerhans cells and interstitial dendritic cells. In the lymph and blood, they include afferent lymph veiled cells and blood dendritic cells, respectively. In lymphoid organs, they include lymphoid dendritic cells and interdigitating cells.

As used herein, "modified" to present an epitope refers to antigen-presenting cells (APCs) that have been manipulated to present an epitope by natural or recombinant methods. For example, the APCs can be modified by exposure to the isolated antigen, alone or as part of a mixture, peptide loading, or by genetically modifying the APC to express a polypeptide that includes one or more epitopes.

As used herein, "pharmaceutically acceptable salt" refers to a salt that retains the desired biological activity of the parent compound and does not impart any undesired toxicological effects. Examples of such salts include, but are not limited to, (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, furmaric acid, gluconic acid, citric acid, malic acid, ascorbic acid, benzoic acid, tannic acid, pamoic acid, alginic acid, polyglutamic acid, naphthalenesulfonic acids, naphthalenedisulfonic acids, polygalacturonic acid; (b) salts with polyvalent metal cations such as zinc, calcium, bismuth, barium, magnesium, aluminum, copper, cobalt, nickel, cadmium, and the like; or (c) salts formed with an organic cation formed from N,N'-dibenzylethylenediamine or ethylenediamine; or (d) combinations of (a) and (b) or (c), e.g., a zinc tannate salt; and the like. The preferred acid addition salts are the trifluoroacetate salt and the acetate salt.

As used herein, "pharmaceutically acceptable carrier" includes any material which, when combined with an active ingredient, allows the ingredient to retain biological activity and is non-reactive with the subject's immune system. Examples include, but are not limited to, any of the standard pharmaceutical carriers such as a phosphate buffered saline solution, water, emulsions such as oil/water emulsion, and various types of wetting agents. Preferred diluents for aerosol or parenteral administration are phosphate buffered saline or normal (0.9%) saline.

Compositions comprising such carriers are formulated by well known conventional methods (see, for example, Remington's Pharmaceutical Sciences, 18th edition, A. Gennaro, ed., Mack Publishing Co., Easton, PA, 1990).

As used herein, "adjuvant" includes those adjuvants commonly used in the art to facilitate the stimulation of an immune response.

As used herein, "a" or "an" means at least one, unless clearly indicated otherwise.

### Encapsulation in Microspheres

While encapsulation of protein and/or adjuvant can be achieved by a variety of methods known in the art, the encapsulation is preferably performed via hydrophobic ion pairing as described herein. Other microsphere encapsulation methods are described in WO02/03961 (PCT/US01/21780, filed July 9, 2001) and PCT/US02/00235, filed January 7, 2002.

The invention provides a method for encapsulating a protein and/or adjuvant into microspheres via hydrophobic ion pairing (HIP). The method comprises extracting an aqueous solution comprising the protein with an organic solvent containing a HIP agent to produce an extraction product having an organic phase comprising the protein. The method further comprises recovering the organic phase from the extraction product, and dissolving a polymer in the aqueous solution or in the organic phase. Microspheres are then prepared from a polymer solution, wherein the polymer solution comprises the recovered organic phase, the protein, and the polymer. Hydrophobic ion pairing allows extraction of protein into an organic medium, thereby allowing microsphere formulations to be prepared with a single emulsion. The resulting microspheres display desirable release kinetics, i.e., low initial burst and controlled release of the protein over time.

In one embodiment, the HIP agent is an anionic HIP agent, such as docusate sodium, and the HIP agent is present in stoichiometric amounts equal to or greater than the number of net positive charges on the protein. In another embodiment, the HIP agent is a cationic HIP agent, such as dimethyldioctadecyl-ammonium bromide (DDAB18); 1,2-dioleoyloxy-3-(trimethylammonium)propane (DOTAP); or cetrimonium bromide (CTAB). In this embodiment, the HIP agent is present in stoichiometric amounts equal to or greater than the number of net negative charges on the protein. Preferably, the organic medium has a ratio of HIP agent to protein of up to about 70:1.

The HIP agent and the aqueous solution are selected in accordance with the characteristics of the protein to be encapsulated. Typically, for proteins having an isoelectric point (pI) at or below 7.0, an anionic HIP agent is preferred. Likewise, for proteins having a pI greater than or equal to 7.0, a cationic HIP agent is preferred. For encapsulation of a protein having a pI of about 7.0, either a cationic or anionic HIP agent can be used. The pH of the aqueous solution can be adjusted to achieve the appropriate charge characteristics. Typically, aqueous solutions having low salt concentrations are preferred. In one embodiment, the aqueous solution has a total salt concentration of less than about 30 mM. The method is suitable for proteins having a variety of isoelectric points, including those having a pI of at least about 7.5, at least about 8.0, up to about 6.0, up to about 6.5, as well as pI's of up to or greater than about 7.0.

Typically, a protein solution containing low concentrations of calcium chloride and other salts (preferably less than 30 mM total) is adjusted to a pH of 3 to 5 (preferably at least two pH units below the pI of the protein). The aqueous protein solution is then extracted with an organic medium containing an HIP agent, such as docusate sodium. The docusate sodium is present in stoichiometric amounts equal to or greater than the number of positive charges on the protein. The organic phase can be recovered from the extraction by centrifugation. A polymer and additives that are soluble in an organic solvent, such as methylene chloride, are then dissolved in the organic phase with the protein. Although it is more practical to dissolve the polymer in the organic phase, the polymer could also be added to the protein solution prior to the extraction. In addition, the salt concentrations in the protein solution and the concentration of the HIP agent can be varied to obtain cleaner separation of the two phases upon centrifugation. Likewise, the pH can be optimized for a given protein.

Examples of organic solvents suitable for use with the method of the invention include, but are not limited to, methylene chloride, dichloromethane, chloroform, ethylacetate, or dimethylsulfoxide. Methylene chloride is preferred.

The microspheres can be prepared by a variety of methods known in the art, including a single oil-in-water emulsion, a double oil-in-water emulsion, spray drying or coacervation of the polymer solution. An advantage of the method of the invention, is that it allows for preparation of the microspheres by a single emulsion, and thereby obtaining microspheres exhibiting improved release kinetics.

Typically, the method of the invention will result in the formation of microspheres of a suitable size for administration and delivery of proteins, particularly as vaccines. Preferably, at least about 90% of the microspheres are about 1 to about 10 µm in diameter.

The microspheres of the invention preferably comprise a biodegradable polymer, such as poly(lacto-co-glycolide) (PLG), poly(lactide), poly(caprolactone), poly(hydroxybutyrate) and/or copolymers thereof. Alternatively, the microspheres can comprise another wall-forming material. Suitable wall-forming materials include, but are not limited to, poly(dienes) such as poly(butadiene) and the like; poly(alkenes) such as polyethylene, polypropylene, and the like; poly(acrylics) such as poly(acrylic acid) and the like; poly(methacrylics) such as poly(methyl methacrylate), poly(hydroxyethyl methacrylate), and the like; poly(vinyl ethers); poly(vinyl alcohols); poly(vinyl ketones); poly(vinyl halides) such as poly(vinyl chloride) and the like;, poly(vinyl nitriles), poly(vinyl esters) such as poly(vinyl acetate) and the like; poly(vinyl pyridines) such as poly(2-vinyl pyridine), poly(5-methyl-2-vinyl pyridine) and the like; poly(styrenes); poly(carbonates); poly(esters); poly(orthoesters); poly(esteramides); poly(anhydrides); poly(urethanes); poly(amides); cellulose ethers such as methyl cellulose, hydroxyethyl cellulose, hydroxypropyl methyl cellulose, and the like; cellulose esters such as cellulose acetate, cellulose acetate phthalate, cellulose acetate butyrate, and the like; poly(saccharides), proteins, gelatin, starch, gums, resins, and the like. These materials may be used alone, as physical mixtures (blends), or as copolymers. Biodegradable microspheres (e.g., polylactate polyglycolate) for use as carriers are disclosed, for example, in U.S. Patent Nos. 4,897,268; 5,075,109; 5,928,647; 5,811,128; 5,820,883; 5,853,763; 5,814,344; 5,407,609; and 5,942,252; the disclosures of each of which are incorporated herein by reference.

In a preferred embodiment, the polymer comprises PLG. In some embodiments, the PLG can include ester end groups or carboxylic acid end groups, and have a molecular weight of from about 4 kDa to about 120 kDa, or preferably, about 8 kDa to about 65 kDa.

Preferably, the polymer solution further comprises an adjuvant. Examples of adjuvants include, but are not limited to, helper peptide; aluminum salts such as aluminum hydroxide gel (alum) or aluminum phosphate; Freund's Incomplete Adjuvant and Complete Adjuvant (Difco Laboratories, Detroit, MI); Merck Adjuvant 65 (Merck and Company, Inc., Rahway, NJ); AS-2 (Smith-Kline Beecham); QS-21 (Aquilla); MPL™ immunostimulant or 3d-MPL (Corixa Corporation); LEIF; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A; muramyl tripeptide phosphatidyl ethanolamine or an immunostimulating complex, including cytokines (e.g., GM-CSF or interleukin-2, -7 or -12) and immunostimulatory DNA sequences. Preferred adjuvants include MPL, saponin, aluminum phosphate, calcium phosphate, aminoalkylglucosaminide phosphate, isotucerasol, cell wall skeleton, and/or a CpG-containing oligonucleotide.

The release rate of the microspheres will be influenced by the properties of the buffer used. For example, HEPES buffer will result in slower release than Tris buffer. In addition, the incorporation of fatty acid esters and cholesterol into microspheres to modify the release kinetics of encapsulated drug has been described by Urata et al., 1999, J. Controlled Release 58:133-141, and these principles can be adapted for use with encapsulated proteins. Examples of fatty acid esters include, but are not limited to, ethyl myristate (C14), ethyl caprate (C10) and ethyl stearate (C18).

The protein to be encapsulated in the microspheres typically comprises an antigen, such as an antigen associated with cancer, autoimmune disease or an infectious disease. Examples of cancer include, but are not limited to, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oliodendroglioma, meningioma, melanoma, neuroblastoma, retinoblastoma, leukemia, lymphoma, multiple myeloma, Waldenström's macroglobulinemia, and heavy chain disease. An example of a cancer antigen is her2/neu, a breast cancer antigen (Bargmann et al., 1986, Nature 319(6050):226-30; Bargmann et al., 1986, Cell 45(5):649-57). Examples of her-2/neu antigens include, but are not limited to, the intracellular domain of her-2/neu (ICD, amino acid residues 676-1255; see Bargmann et al. references above), p369 (also known as E75; KIFGSLAFL; SEQ ID NO: 1) of the extracellular domain of her-2/neu, ECD-PD (fusion of extracellular domain and phosphorylated portion of the intracellular domain; see WO02/123,41, published February 14, 2002, and WO00/44899, published August 3, 2000), and p546, a transmembrane region of her-2/neu (VLQGLPREYV; SEQ ID NO: 2).

Examples of infectious disease include, but are not limited to, infection with a pathogen, virus, bacterium, fungus or parasite. Examples of viruses include, but are not limited to, hepatitis type B or type C, influenza, varicella, adenovirus, herpes simplex virus type I or type II, rinderpest, rhinovirus, echovirus, rotavirus, respiratory syncytial virus, papilloma virus, papova virus, cytomegalovirus, echinovirus, arbovirus, hantavirus, coxsachie virus, mumps virus, measles virus, rubella virus, polio virus, human immunodeficiency virus type I or type II. Examples of bacteria include, but are not limited to, *M. tuberculosis,* mycobacterium, mycoplasma, neisseria and legionella. Examples of parasites include, but are not limited to, rickettsia and chlamydia.

In one embodiment, the infectious disease is tuberculosis. Representative tuberculosis antigens include Mtb8.4, TbH9 (Mtb 39A), 38-1, Mtb41, Mtb40, Mtb32A, Mtb9.9A, Mtb9.8, (Mtb16, Mtb72f, Mtb59f, Mtb88f, Mtb71f, Mtb46f and Mtb31f. The "f' indicates a fusion or two or more proteins.

### Compositions

The invention provides compositions that are useful for delivering proteins and/or adjuvants. The proteins encapsulated in microspheres can include antigens associated with cancer, autoimmune disease or infectious disease, providing compositions for treating and preventing cancer or infectious disease. In one embodiment, the composition is a pharmaceutical composition. The composition can comprise a therapeutically or prophylactically effective amount of a protein that includes one or more antigens associated with cancer, autoimmune disease, or infectious disease. An effective amount is an amount sufficient to elicit or augment an immune response, e.g., by activating T cells. One measure of the activation of T cells is a cytotoxicity assay or an interferon-gamma release assay, as described in the examples below. In some embodiments, the composition is a vaccine.

The composition can optionally include a carrier, such as a pharmaceutically acceptable carrier. Pharmaceutically acceptable carriers are determined in part by the particular composition being administered, as well as by the particular method used to administer the composition. Accordingly, there is a wide variety of suitable formulations of pharmaceutical compositions of the present invention. Formulations suitable for parenteral administration, such as, for example, by intraarticular (in the joints), intravenous, intramuscular, intradermal, intraperitoneal, and subcutaneous routes, and carriers include aqueous isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, preservatives and emulsions.

The composition of the invention can further, or alternatively, comprise one or more adjuvants. Adjuvant can be encapsulated in microspheres, together with or separately from protein. Alternatively, or in addition, adjuvant can be provided in the composition using a conventional formulation rather than encapsulation in microspheres. Examples of adjuvants include, but are not limited to, helper peptide, alum, Freund's, muramyl tripeptide phosphatidyl ethanolamine or an immunostimulating complex, including cytokines. Preferred adjuvants include MPL, saponin, AS-2, aluminum phosphate, calcium phosphate, aminoalkylglucosaminide phosphate, isotucerasol, cell wall skeleton, and/or a CpG-containing oligonucleotide.

Most adjuvants contain a substance designed to protect the antigen from rapid catabolism, such as aluminum hydroxide or mineral oil, and a stimulator of immune responses, such as lipid A, *Bortedella pertussis* or *Mycobacterium tuberculosis* derived proteins. Suitable adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); aluminum salts such as aluminum hydroxide gel (alum) or aluminum phosphate; salts of calcium, iron or zinc; an insoluble suspension of acylated tyrosine acylated sugars; cationically or anionically derivatized polysaccharides; polyphosphazenes biodegradable microspheres; monophosphoryl lipid A and quil A. Cytokines, such as GM CSF or interleukin-2, -7, or -12, may also be used as adjuvants.

Within the vaccines provided herein, the adjuvant composition is preferably designed to induce an immune response predominantly of the Th1 type. High levels of Th1-type cytokines (e.g., IFN-γ, IL-2 and IL-12) tend to favor the induction of cell mediated immune responses to an administered antigen. In contrast, high levels of Th2-type cytokines (e.g., IL-4, IL-5, IL-6, IL-10 and TNF-β) tend to favor the induction of humoral immune responses. Following application of a vaccine as provided herein, a patient will support an immune response that includes Th1- and Th2-type responses. Within a preferred embodiment, in which a response is predominantly Th1-type, the level of Thl-type cytokines will increase to a greater extent than the level of Th2-type cytokines. The levels of these cytokines may be readily assessed using standard assays. For a review of the families of cytokines, see Mosmann and Coffman, 1989, Ann. Rev. Immunol. 7:145-173.

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Corixa Corporation (Hamilton, MT) (see US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555. Another preferred adjuvant is a saponin, preferably QS21, which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprises an oil-in-water emulsion and tocopherol. A particularly potent adjuvant formulation involving QS21, 3D-MPL and tocopherol in an oil-in-water emulsion is described in WO 95/17210. Another adjuvant that may be used is AS-2 (Smith-Kline Beecham). Any vaccine provided herein may be prepared using well known methods that result in a combination of antigen, immune response enhancer and a suitable carrier or excipient.

Vaccine preparation is generally described in, for example, M.F. Powell and M.J. Newman, eds., "Vaccine Design (the subunit and adjuvant approach)," Plenum Press (NY, 1995). Pharmaceutical compositions and vaccines within the scope of the present invention may also contain other compounds, which may be biologically active or inactive.

Such compositions may also comprise buffers (e.g., neutral buffered saline or phosphate buffered saline), carbohydrates (e.g., glucose, mannose, sucrose or dextrans), mannitol, proteins, polypeptides or amino acids such as glycine, antioxidants, chelating agents such as EDTA or glutathione, adjuvants (e.g., aluminum hydroxide) and/or preservatives. Alternatively, compositions of the present invention may be formulated as a lyophilizate.

The compositions described herein may be administered as part of a sustained release formulation (i.e., a formulation such as a capsule or sponge that effects a slow release of compound following administration). Such formulations may generally be prepared using well known technology and administered by, for example, oral, rectal or subcutaneous implantation, or by implantation at the desired target site. Sustained-release formulations may contain a polypeptide or protein dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

### Methods

The invention also provides methods for delivering a protein or an adjuvant to a subject, for eliciting an immune response to an antigen in a subject, and for treating or preventing a condition in a subject. These methods comprise administering to the subject a composition of the invention. Administration may be performed as described below.

In some embodiments, the condition to be treated or prevented is cancer or a precancerous condition (e.g., hyperplasia, metaplasia, dysplasia). Examples of cancer are listed hereinabove. In some embodiments, the condition to be treated or prevented is an autoimmune disease or infectious disease. Examples of infectious disease include the viral, bacterial and parasitic diseases described hereinabove. One example of an infectious disease is tuberculosis. Examples of autoimmune disease include allergy, insulin-dependent diabetes mellitus, systemic lupus erythematosus, pernicious anemia, Hashimoto's thyroiditis, Addison's disease, dermatomyositis, and rheumatoid arthritis.

### Administration of the Compositions

Treatment includes prophylaxis and therapy. Prophylaxis or treatment can be accomplished by a single direct injection at a single time point or multiple time points. Administration can also be nearly simultaneous to multiple sites. Patients or subjects include mammals, such as human, bovine, equine, canine, feline, porcine, and ovine animals. Preferably, the patients or subjects are human.

Compositions are typically administered *in vivo* via parenteral (e.g. intravenous, subcutaneous, and intramuscular) or other traditional direct routes, such as buccal/sublingual, rectal, oral, nasal, topical, (such as transdermal and ophthalmic), vaginal, pulmonary, intraarterial, intraperitoneal, intraocular, or intranasal routes or directly into a specific tissue. Intramuscular administration is preferred.

The dose administered to a patient, in the context of the present invention should be sufficient to effect a beneficial therapeutic response in the patient over time, or to inhibit infection or disease due to infection. Thus, the composition is administered to a patient in an amount sufficient to elicit an effective immune response to the specific antigens and/or to alleviate, reduce, cure or at least partially arrest or prevent symptoms and/or complications from the disease or infection. An amount adequate to accomplish this is defined as a "therapeutically effective dose."

The dose will be determined by the activity of the composition produced and the condition of the patient, as well as the body weight or surface areas of the patient to be treated. The size of the dose also will be determined by the existence, nature, and extent of any adverse side effects that accompany the administration of a particular composition in a particular patient. In determining the effective amount of the composition to be administered in the treatment or prophylaxis of diseases, the physician needs to evaluate the production of an immune response against the pathogen, progression of the disease, and any treatment-related toxicity.

Compositions comprising immune cells are preferably prepared from immune cells obtained from the subject to whom the composition will be administered. Alternatively, the immune cells can be prepared from an HLA-compatible donor. The immune cells are obtained from the subject or donor using conventional techniques known in the art, exposed to APCs modified to present an epitope of the invention, expanded *ex vivo,* and administered to the subject. Protocols for *ex vivo* therapy are described in Rosenberg et al., 1990, New England J. Med. 9:570-578.

Immune cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokine (such as IL-2) and non-dividing feeder cells. As noted above, immunoreactive polypeptides as provided herein may be used to enrich and rapidly expand antigen-specific T cell cultures in order to generate a sufficient number of cells for immunotherapy. In particular, antigen-presenting cells, such as dendritic, macrophage, monocyte, fibroblast and/or B cells, may be pulsed with immunoreactive polypeptides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow *in vivo* and to survive long term in substantial numbers by repeated stimulation with antigen supplemented with IL-2 (see, for example, Cheever et al., 1997, Immunological Reviews 157:177).

Administration by many of the routes of administration described herein or otherwise known in the art may be accomplished simply by direct administration using a needle, catheter or related device, at a single time point or at multiple time points.

### Antigen-Presenting Cells

A composition of the invention may be employed to facilitate production of an antigen-specific immune response that targets cancerous or infected cells. Certain preferred embodiments of the present invention use dendritic cells or progenitors thereof as antigen-presenting cells (APCs). Dendritic cells are highly potent APCs (Banchereau and Steinman, Nature 392:245-251, 1998) and have been shown to be effective as a physiological adjuvant for eliciting prophylactic or therapeutic immunity (see Timmerman and Levy, Ann. Rev. Med. 50:507-529, 1999). In general, dendritic cells may be identified based on their typical shape (stellate *in situ,* with marked cytoplasmic processes (dendrites) visible *in vitro*) and based on the lack of differentiation markers of B cells (CD19 and CD20), T cells (CD3), monocytes (CD14) and natural killer cells (CD56), as determined using standard assays. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine (Zitvogel et al., 1998, Nature Med. 4:594-600).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor, mannose receptor and DEC-205 marker. The mature phenotype is typically characterized by a lower expression of these markers, but a high expression of cell surface molecules responsible for T cell activation such as class I and class II MHC, adhesion molecules (e.g., CD54 and CD11) and costimulatory molecules (e.g., CD40, CD80 and CD86). APCs may be combined with a protein encapsulated in a microsphere of the invention such that the APCs can take up and express the polypeptide, or an immunogenic portion thereof, which is expressed on the cell surface. Antigen loading of dendritic cells may be achieved by incubating dendritic cells or progenitor cells with the encapsulated protein. A dendritic cell may be pulsed with an immunological partner that provides T cell help (e.g., a carrier molecule).

### EXAMPLES

The following examples are presented to illustrate the present invention and to assist one of ordinary skill in making and using the same. The examples are not intended in any way to otherwise limit the scope of the invention.

### Example 1: Protein-Microsphere Formulations

This example describes the preparation of protein-microsphere and protein-adjuvant-microsphere formulations.

### MJ071b and MJ087b

These microsphere formulations were prepared, in the absence of adjuvant, using a hydrophobic ion pair (HIP) technique. 3 mg of lyophilized protein was dissolved in 2.7 ml of ultra-pure water. To this protein solution was added 0.3 ml of a 100 mM CaCl₂ solution and 55 µl of 0.1 M HCl, to lower the pH into the pH 3-5 range. The protein was extracted into the organic phase, 4.3 mM AOT (docusate sodium) in dichloromethane, by vortex mixing. The organic phase, containing the protein, was separated from the aqueous phase by centrifugation. The aqueous phase was discarded and the volume of organic was brought up to 10 ml through the addition of DCM. PLG polymer (300 mg of RG502H; Boehringer Ingelheim GmbH (Ingelheim, Germany)) was then dissolved in the solvent. Formation of the microspheres was achieved through the addition of the protein and polymer in organic phase to 400 ml of a 5% PVA (polyvinyl alcohol) aqueous solution with a Silvers on mixer at 9000 rpm for approximately 1 minute. The microsphere were stirred gently for 2-3 hours to allow hardening and then washed and collected by centrifugation. Manntitol was added prior to freezing and lyophilization as an excipient.

### MJ082

These microspheres contained both protein (Mtb8.4; also referred herein as DPV) and adjuvant (MPL). During co-encapsulation, the ratio of protein to MPL was fixed at ∼1:1. This formulation was prepared in the same manner as MJ071b/MJ087b with the sole exception that 3 mg of MPL was added to the organic phase after protein extraction and before addition to the process media (*i.e.* 400 ml of 5% PVA).

### MJ083

These microspheres contained both protein (Mtb8.4) and adjuvant (MPL). As they were co-encapsulated, the ratio of protein to MPL was fixed and was ∼1:1. This formulation was prepared in the same general manner as MJ071b/MJ087b, with the exception of the MPL addition. The MPL (3 mg) was incorporated via an inner aqueous phase. That is, 3 mg of MPL was first dispersed in 1.0 ml of water with aggressive vortex mixing. After addition of PLG polymer to the DCM containing protein phase, this 1.0 ml of MPL in water phase was added and emulsified by vortex mixing for 30 seconds. This primary emulsion was then emulsified in 400 ml of 5% PVA as before.

### MJ084

These microspheres contained both protein (Mtb8.4) and adjuvant (MPL). The ratio of protein to MPL was fixed and was 0.37. This formulation was prepared in the same manner as MJ071b/MJ087b with the sole exception that 3 mg of MPL was added to the 400 ml of process media in place of the PVA, the emulsion was mixed at 9000 rpm for 2-1/2 minutes after which the dispersion was further diluted by adding an additional 300 ml of ultra-pure water.

### Microsphere Properties

| Lot # | rDPV Core-Loading | MPL Core-Loading | Size (µm) (by SEM) | rDPV:MPL Ratio | MPL (µg) per 12 µg rDPV |
|---|---|---|---|---|---|
| MJ071b | 0.54% | -- | 0.8 | -- | -- |
| MJ082 | 0.68% | 0.65% | 0.8 | 1.0 | 11.5 |
| MJ083 | 0.56% | 0.58% | 0.8 | 1.0 | 12 |
| MJ084 | 0.16% | 0.43% | 0.8 | 0.37 | 32 |

### Example 2: Release of DPV (Mtb8.4) Protein from HIP Microspheres

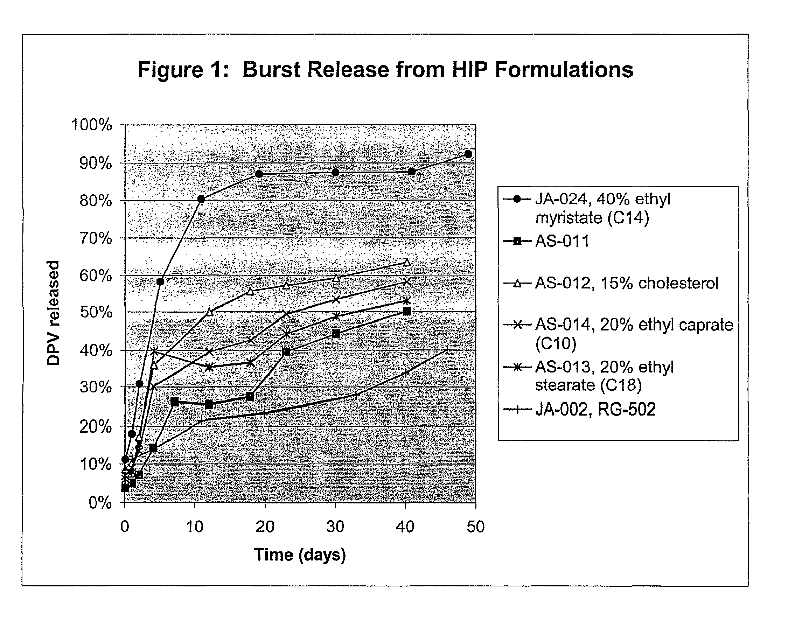

The example shows the *in vitro* release of DPV protein into a release medium composed of 150 mM Tris, pH 8.1, and 0.01% Tween 20. DPV is a 9 kilodalton protein with a pI of 6.5. Figure 1 shows DPV release plotted as a function of time for five microsphere formulations. The formulations included JA-024, 40% ethyl myristate (C14) (diamonds); AS-011 (squares); AS-012, 15% cholesterol (triangles); AS-014, 20% ethyl caprate (C10) (X's); AS-013, 20% ethyl stearate (C18) (asterisks); and JA-002, RG-502 (+'s).

The formulations were each prepared by a single emulsion method in which the DPV protein was solubilized in methylene chloride via hydrophobic ion pairing (HIP) with docusate sodium. Formulation AS-011 through AS-014 were prepared using PLG RG-592H polymer. JA=002 was prepared with PLG RG-502, an endcapped polymer which is more hydrophobic than RG-502H. Cholesterol and fatty acid esters were included in some embodiments, as noted above.

### Example 3: Mtb8.4 Antigen Encapsulated in HIP Microspheres Elicits Strong CTL Responses in Mice

This example shows that Mtb8.4 protein microspheres prepared using a HIP technique elicited stronger CTL responses than did a potent adjuvant combination and protein alone. The CTL responses for individual mice using a chromium release assay after one *in vitro* stimulation of mouse splenocytes are shown in Figures 2A-C. Mice that were immunized with microencapsulated protein (Fig. 2A) elicited the strongest, most consistent immune responses, compared with the responses elicited by protein plus MPL/saponin (Fig. 2B) and protein alone (Fig. 2C). Mice were immunized on D0 and D21 with 5 µg of protein subcutaneously. Spleens were harvested on D35. No specific lysis was observed for naïve mice. The Mtb8.4 protein-microspheres elicited stronger and more consistent CTL responses than either the protein plus MPL/saponin group or the protein alone group (Fig. 2A-C).

### Example 4: HIP Microspheres Elicit Strong Antibody Responses in Mice

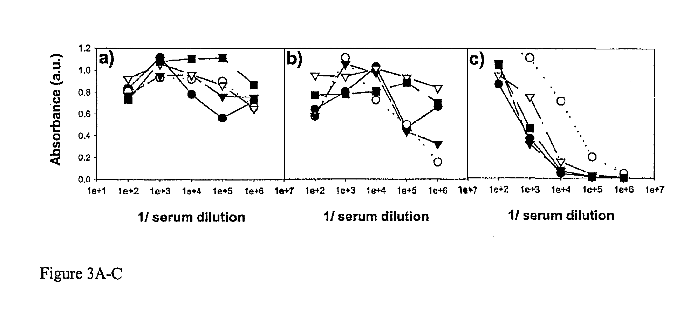

This example describes an experiment in which antibody responses (IgG1) were measured from sera obtained on the day of harvest. As shown in Figures 3A-C, the protein microspheres prepared using the HIP technique of the invention elicited a strong antibody response in all five mice studied (Fig. 3A). These responses were significantly stronger than those elicited by protein alone (Fig. 3B) and equivalent to those elicited by the MPL/saponin adjuvant combination (Fig. 3C). Mice, in groups of five, were immunized on D0 and D21 with 5 µg of protein subcutaneously. Sera were collected on D35 and specific antibody levels were measured by ELISA. The results from individual mice are shown.

### Example 5: HIP Microspheres Elicit Stronger and More Consistent Immune Responses in Mice Than Immunization With DNA or Protein/Adjuvant Combination

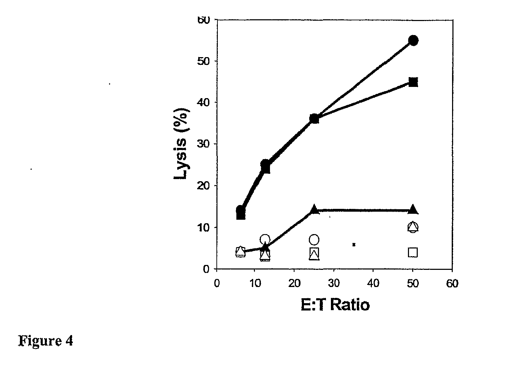

This example provides further evidence that protein-microsphere formulations of the invention are effective at eliciting immune responses. Figure 4 shows the results from a mouse experiment that compared the ability of a Mtb8.4 protein-microsphere to elicit CTL responses with those elicited by DNA immunizations and protein plus the MPL/saponin adjuvant combination. CTL responses were measured for groups of mice from pooled spleens (n=7) using a chromium release assay after one *in vitro* stimulation of mouse splenocytes. Mice were immunized on D0 and D21 with 15 µg of protein subcutaneously or 50 µg of DNA intramuscularly. Spleens were harvested on D43. Closed symbols represent lysis of targets transduced with Mtb8.4 DNA. Open symbols represents lysis of control EL4 targets. As shown in Figure 4, strong CTL response was measured for the group receiving protein-microspheres (circles). This response was significantly stronger than the response measured for the protein plus MPL/saponin group (triangles) and, remarkably, comparable to the response for the group that received DNA (squares).

The antibody results from this experiment showed that the protein-microspheres and protein plus MPL/saponin adjuvant combination elicited antibody responses that were similar in strength, while the DNA immunized group elicited no detectable antibody responses. The inability of DNA vaccines to elicit strong antibody responses is a key reason for including protein-microspheres in, for example, an HIV vaccine designed to elicit neutralizing antibodies.

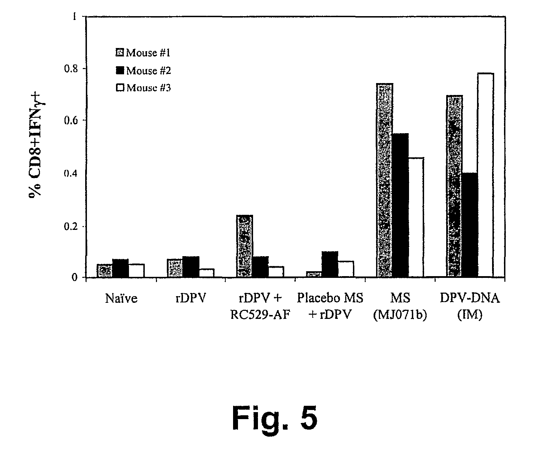

Figures 5-7 show ICC, ELISA and CTL data, respectively, for C57BL/6 mice immunized one or two times with 12 µg of Mtb8.4-microspheres or protein + adjuvant. Vaccines were given via subcutaneous administration with 21 days between the primary and secondary immunizations. For Mtb8.4-DNA immunizations, mice were administered 50 µg of plasmid DNA via intramuscular injection. Mice were harvested at various times post secondary immunizations and analyzed for anti-Mtb8.4 immune responses.
1. CTL Assay: Specific lysis of Mtb8.4-EL4 targets by CD8+ T-cells.
2. ICC Assay: Intracellular cytokine staining of CD8+ and CD4+ T-cells following a 5 hr activation with Mtb8.4 peptides.
3. IFNγ ELISA: IFNγ release from rMtb8.4 activated spleen cell cultures.
4. Anti-Mtb8.4 serum IgG₁ and IgG_{2b.}

Figure 5 shows that C57B1/6 mice were administered a single subcutaneous (SC) or intradermal (ID) immunization with recombinant Mtb8.4 protein plus PBS, Mtb8.4 encapsulated in microspheres (MJ-071b), Mtb8.4 protein plus adjuvant (RC-529-AF), or Mtb8.4 surface adsorbed microspheres (AM-008). These data indicate the percentage of CD8+IFNγ+ cells (by ICC) in the spleens of individual mice 14 days following a single SC or ID immunization.

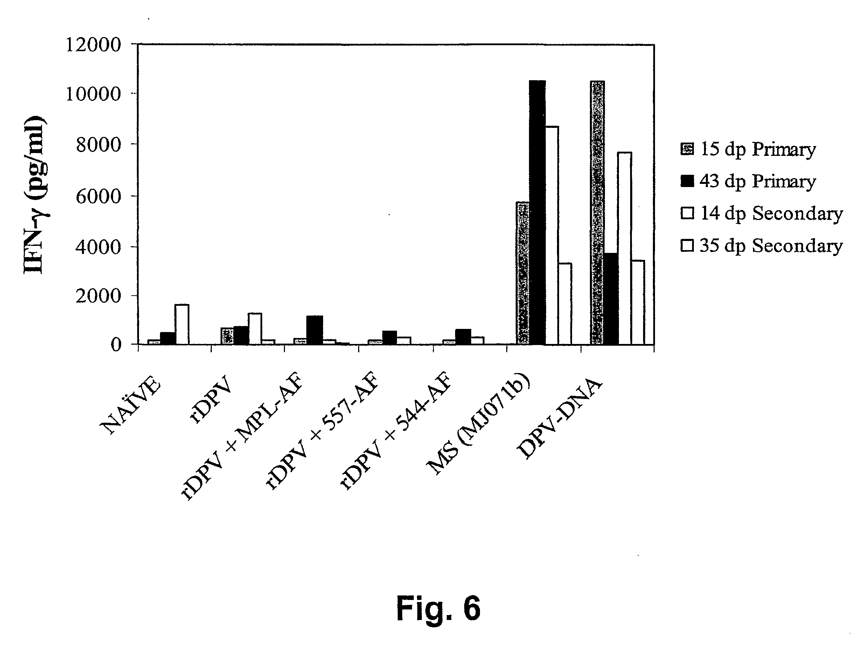

Figure 6 shows that C57B1/6 mice were administered one or two subcutaneous (SC) immunizations with recombinant Mtb8.4 protein plus PBS, Mtb8.4 encapsulated in microspheres (MJ-071b), Mt8.4 protein + adjuvant (MPL-AF, 557-AF, 544-AF), or Mtb8.4 DNA (intramuscular immunization). These data show IFNγ release (ELISA) from spleen cell cultures (pooled spleens from 3 mice) activated with recombinant Mtb8.4 for 72 hours.

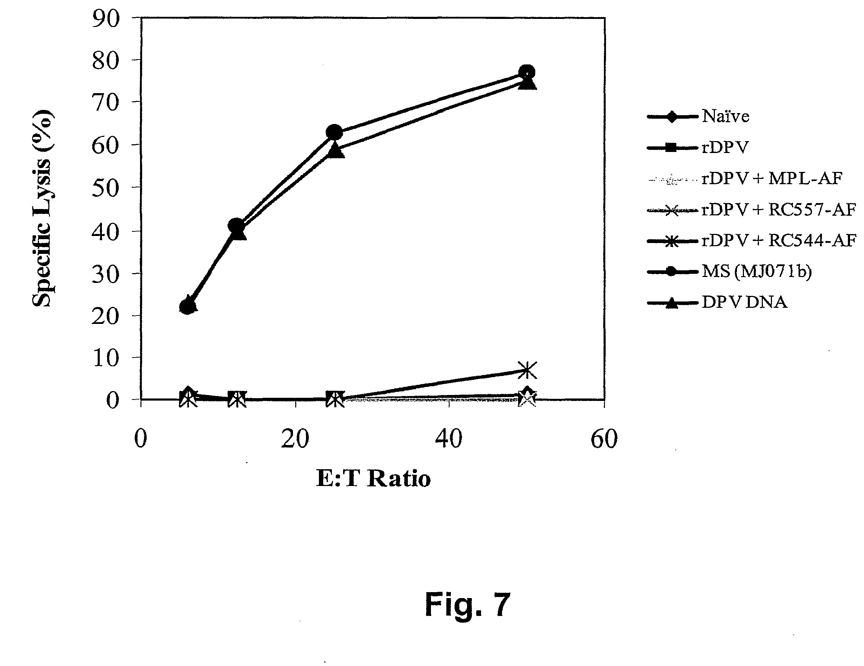

Figure 7 shows that C57B1/6 mice were administered a single subcutaneous (SC) immunization with recombinant Mtb8.4 protein plus PBS, Mtb8.4 encapsulated in microspheres (MJ-071b), Mtb8.4 protein plus adjuvant (MPL-AF, 557-AF, 544-AF), or Mtb8.4 (intramuscular immunization). These data show percentage of specific lysis (CTL assay) of Mtb8.4-EL4 targets by CD8+ T cells following a 5 day activation with irradiated Mtb8.4-EL4 cells.

These data demonstrate the efficacy of a single dose protein-microsphere vaccine for the induction of both cellular and humoral immune responses against the *Mycobacterium tuberculosis* antigen Mtb8.4. Strong anti-Mtb8.4 CD8 T-cell responses were detected following a primary immunization with Mtb8.4-microspheres using both intracellular cytokine staining and specific lysis of Mtb8.4-microspheres using both intracellular cytokine staining and specific lysis of Mtb8.4 expressing targets (CTL assay).

### Example 6: Co-encapsulated Mtb8.4 Protein and Adjuvant (MPL) in Microspheres Induces a Synergistic Response Enhancing Th1 and B-cell Mediated Immunity Against Mtb8.4.

This example demonstrates that the synergistic effect on Th1 and B-cell mediated immunity of co-encapsulation of protein microspheres in the presence of an adjuvant such as MPL.

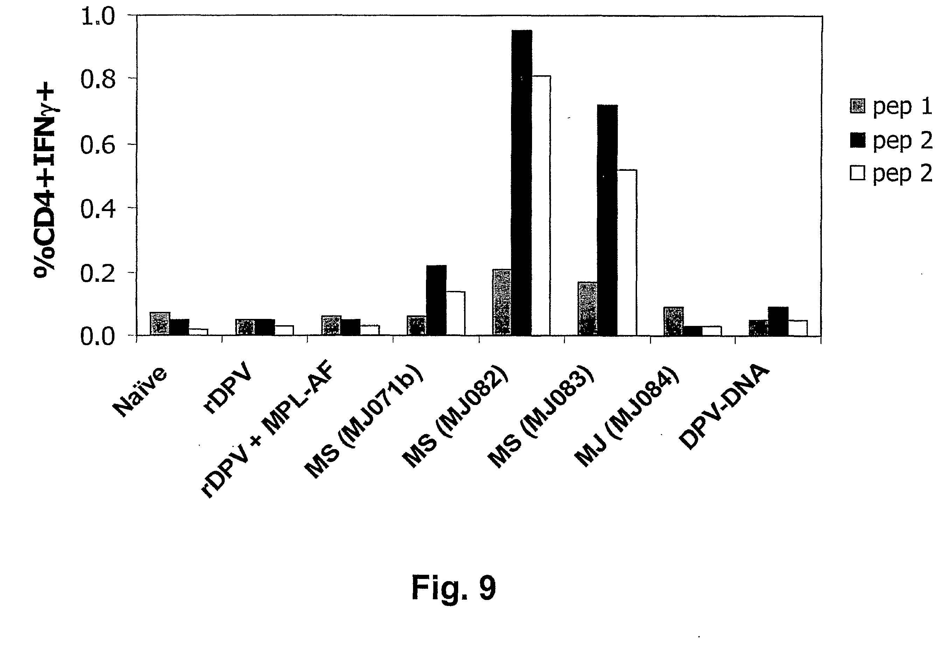

Figures 8 and 9 show the enhancement in CD8 and CD4 T-cell immune responses, respectively, against Mtb8.4 following a single immunization with MPL co-encapsulated microspheres. C57B1/6 mice were immunized (ID) with Mtb8.4-microspheres (MJ-073) or MPL co-encapsulated Mtb8.4-micxospheres prepared using three different methods (MJ-082, MJ-083, or MJ-084). These data show the percentage of Mtb8.4 reactive CD8+ (Figure 8) and CD4+ (Figure 9) cells by ICC for IFNγ at 14 days following a primary immunization.

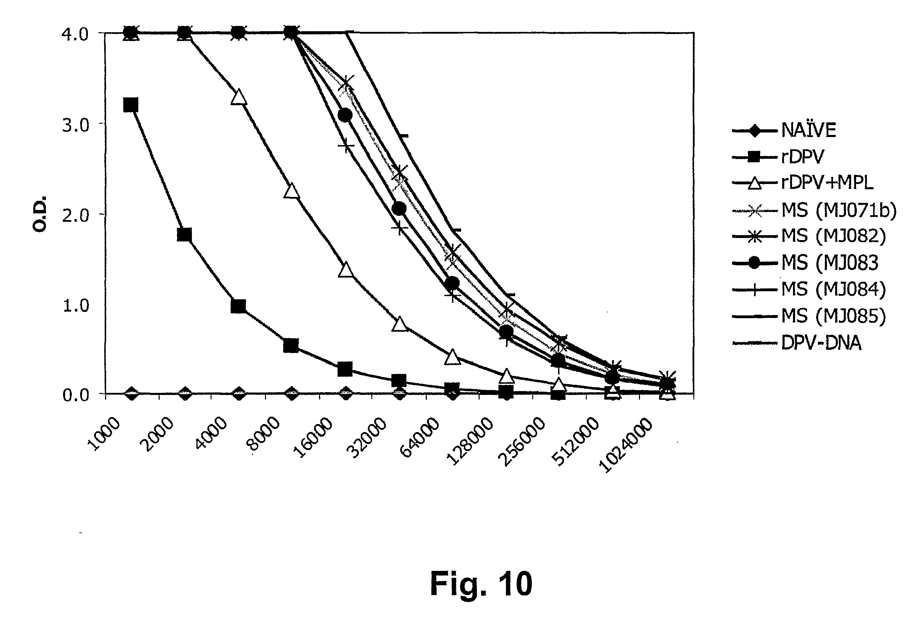

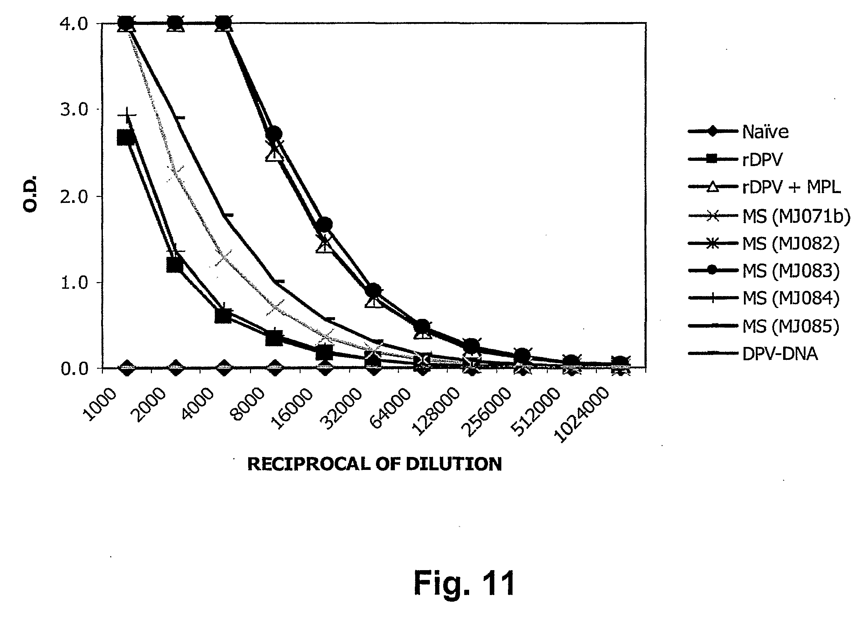

Figures 10 and 11 show the enhancement in IgG₁ and IgG_{2b} antibody responses, respectively, against Mtb8.4 following a single immunization with MPL co-encapsulated microspheres. Serum was collected from C57B1/6 mice 14 days following the secondary immunization with Mtb8.4-microspheres (MJ-073), MPL co-encapsulated Mtb8.4-microspheres (MJ082, MJ083, MJ084) or Mtb8.4 surface adsorbed microspheres (MJ085) and evaluated for anti-Mtb8.4 IgG₁ and IgG_{2b} antibodies (Figures 10 and 11, respectively) by ELISA.

These data demonstrate the synergistic effect on CD4 and CD8 T-cell immune responses when an adjuvant, such as monophosphoryl lipid A (MPL®), is co-encapsulated in the Mtb8.4-microspheres. In addition, and without being limited to a specific mechanistic theory, the MPL containing microspheres significantly enhanced IgG_{2b} serum antibody levels and IFNγ release from spleen cell supernatants indicating that the co-encapsulation of adjuvant, such as MPL, in protein-microspheres results in a polarization towards a Th1 immune response. These data show the efficacy of protein-microspheres as a potent Th1 inducing adjuvant/delivery system for the induction of antigen specific immunity.

### Example 7: Dose Response of Mtb8.4 and MPL Co-Encapsulated Microspheres.

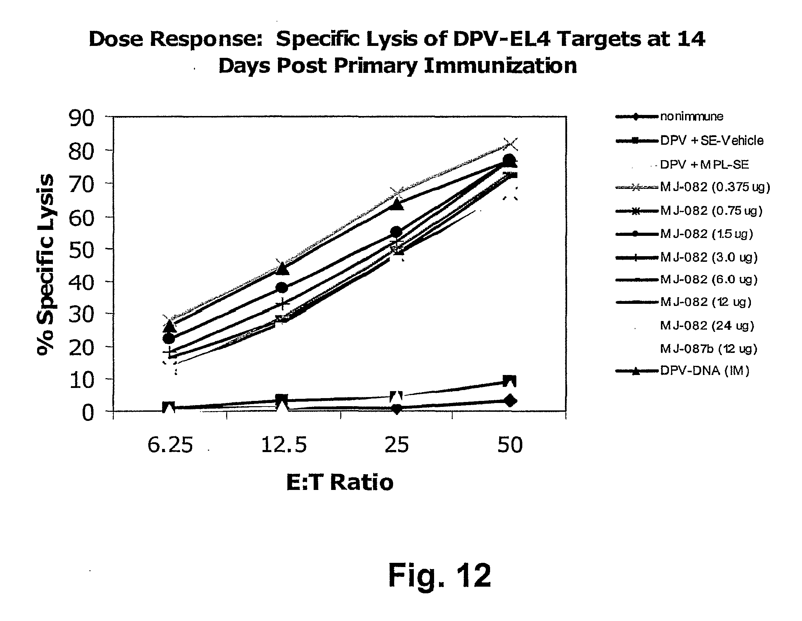

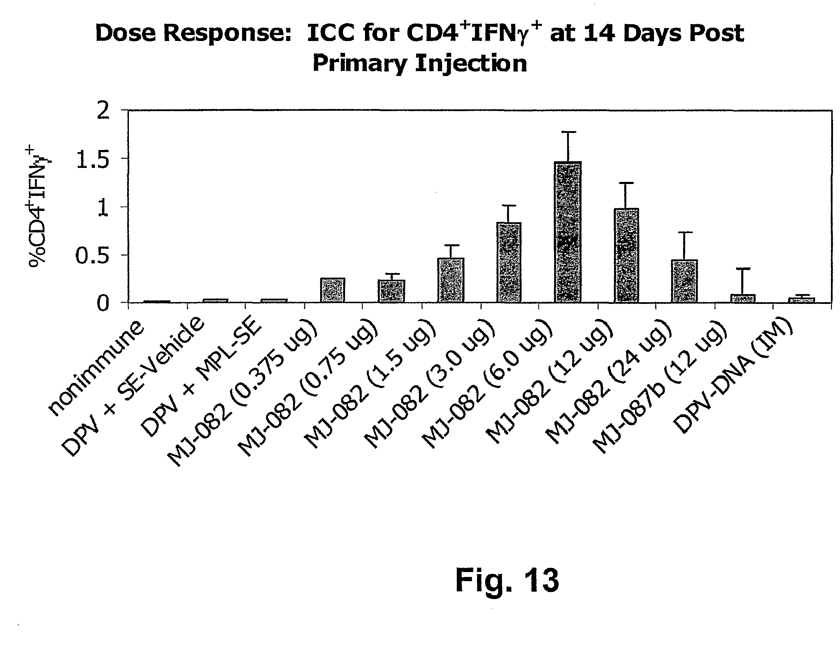

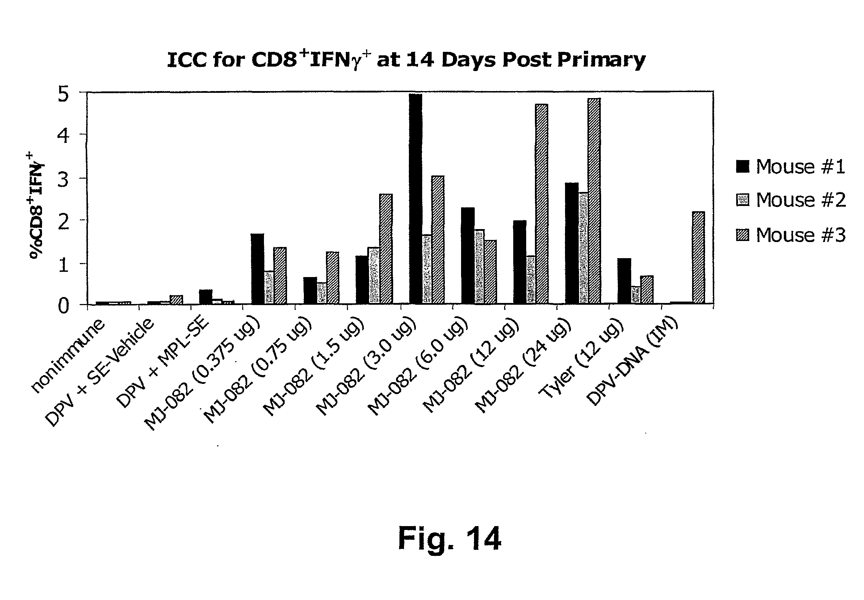

This example discloses the dose response of the MJ-082 protein-MPL co-encapsulation in inducing specific CTL-mediated lysis of Mtb8.4-EL4 target cells, CD4+ T-cell IFNγ release, and CD8+ T-cell IFNγ release. (Figures 12, 13 and 14; respectively). CTL and ICC assays were performed as described elsewhere herein and further support the synergistic effect on CD4 and CD8 T-cell immune responses when an adjuvant, such as monophosphoryl lipid A (MPL®), is co-encapsulated in the Mtb8.4-microspheres.

### Example 8: Optimization of Protein and Adjuvant Combinations

This example demonstrates several aspects of optimal vaccine formulations in accordance with the invention. First, coencapsulation of protein (Mtb8.4) and another adjuvant (RC529) is effective at eliciting CD8+ and CD4+ T-cell responses, CTL, IFN-gamma, and antibody (IgG1 and IgG2a) immune responses. Thus, the comprehensive immune response to coencapsulated protein and adjuvant is not limited to a single adjuvant. Second, encapsulating protein and adjuvant separately and admixing does elicit comprehensive immune responses. Third, having the antigen and the adjuvant encapsulated in the same particles, however, elicits stronger responses than encapsulating in separate particles and admixing. Fourth, adjuvant (MPL or 529) encapsulated in microspheres is at least as effective as adjuvant formulated in the standard aqueous formulation (i.e., liposomes). This novel means of delivering adjuvant, via encapsulation in microspheres, provides an attractive alternative to conventional adjuvant delivery.

Mtb8.4 was used as the protein antigen. RC529, a synthetic aminoalliylglucosaminide phosphate, was used to address the first point, while MPL and 529 were both used to address the subsequent points. Microspheres were prepared containing (a) Mtb8.4 protein alone, (b) MPL® adjuvant alone, (c) RC529 adjuvant alone, (d) Mtb8.4 protein and MPL co-encapsulated, and (e) Mtb8.4 protein and 529 co-encapsulated. Essentially the same method was used to prepare all five types of microspheres with the only difference being in the components that were added.

### Methods

Microsphere Preparation: The same method of microsphere preparation was utilized to prepare the various formulations for this example with the only difference being the components (+/- Mtb8.4 protein, +/-MPL® adjuvant, +/- RC529 adjuvant) that were added. This is the same method that was described in Examples 1, 6, and 7 above. In brief, an aqueous acidic solution was extracted using a dichloromethane solution of docusate sodium. For formulations having protein, the Mtb8.4 protein (3 mg) was in the acid aqueous solution and extracted into the organic phase. Adjuvant (MPL or 529) was then added to the organic phase as required, followed by 300 mg of PLG (RG502H). The organic phase was then emulsified into 400 ml of 5% PVA using a Silverson homogenizer. The solvent was extracted by stirring in a fume-hood for several hours. The microspheres were then washed several times and lyophilized. Mannitol was added prior to lyophilization.

Microsphere Characterization: The protein and adjuvant contents of the microspheres were determined by HPLC or amino acid analysis and the Bartlett inorganic phosphorous assay. Size distributions were measured using a Horiba LA920 and through visual estimations from scanning electron micrographs. Release kinetics were measured by dispersing 10-20 mg of formulation in 100 mM Tris buffer, sample the supernatant over time and quantifying the protein concentration by reverse phase HPLC.

Immunizations: Groups of seven (7) C57B1/6 were immunized as described in Table 8.1. Control groups of mice included naive, Mtb8.4 protein alone, Mtb8.4-DNA, and Mtb8.4 protein-microspheres. There were five test groups for each adjuvant, MPL and 529, which covered the various combinations of reagents. The dose of Mtb8.4 protein was 5 µg. The adjuvant target doses were 5 µg per mouse for MPL or 529. However, the actual amount of adjuvant administered was determined and set by the co-encapsulated microspheres. That is, the adjuvant dose for mice receiving the Mtb8.4-MPL co-encapsulated and Mtb8.4-529 co-encapsulated microspheres were 4.0 and 6.5 µg per mouse, respectively. Hence, all groups receiving MPL received 4.0 µg per mouse and all groups receiving 529 received 6.5 µg per mouse.

**Table 1: Descriptions of Experimental Groups for In Vivo Analysis**

| | | | **Dose (ug per mouse)** | | |
|---|---|---|---|---|---|
| **Group #** | **Name** | **Description** | Mtb8.4 | MPL | 529 |
| | ***Control Groups*** | | | | |
| **A** | Naïve | No immunization | - | - | - |
| **B** | Mtb8.4 | Protein in aqueous solution | 5.0 | - | - |
| **C** | Mtb8.4-DNA | DNA in aqueous solution | 100 | - | - |
| **D** | **(Mtb8.4)-MS** | Protein-microspheres | 5.0 | - | - |
| | | | | | |

| | **MPL** & *Mtb8.4:* | | | | |
|---|---|---|---|---|---|
| **E** | Mtb8.4 + MPL | Protein in aqueos solution admixed with MPL-AF | 5.0 | 4.0 | - |
| **F** | Mtb8.4 + **(MPL)-MS** | Protein in aqueos solution admixed with MPL-microspheres | 5.0 | 4.0 | - |
| **G** | **(Mtb8.4)-MS** + MPL | Protein-microspheres admixed with MPL-AF | 5.0 | 4.0 | - |
| **H** | **(Mtb8.4)-MS + (MPL)-MS** | Protein-microspheres admixed with MPL-microspheres | 5.0 | 4.0 | - |
| **I** | **(Mtb8.4+MPL)-MS** | Protein and MPL co-encapsulated in microspheres | 5.0 | 4.0 | - |
| | | | | | |

| | ***529 & Mtb8.4:*** | | | | |
|---|---|---|---|---|---|
| **J** | Mtb8.4+529 | Protein in aqueos solution admixed with 529-AF | 5.0 | - | 6.5 |
| **K** | Mtb8.4 + **(529)-MS** | Protein in aqueos solution admixed with 529-microspheres | 5.0 | - | 6.5 |
| **L** | **(Mtb8.4)-MS** + 529 | Protein-microspheres admixed with 529-AF | 5.0 | - | 6.5 |
| **M** | **(Mtb8.4)-MS + (529)-MS** | Protein-microspheres admixed with 529-microspheres | 5.0 | - | 6.5 |
| **N** | **(Mtb8.4+529)-MS** | Protein and 529 co-encapsulated in microspheres | 5.0 | - | 6.5 |

| | | | | | |
|---|---|---|---|---|---|
| *Note: The "-AF" formulations ("aqueous formulation") are lipid in Water liposome adjuvant formulations* | | | | | |

All protein formulations were administered by the intradermal route at the base of the tail, while DNA was administered intramuscularly. Three or four mice from each group were harvested two weeks after the primary immunization, with their spleens and sera collected and pooled for evaluation. The remaining three or four mice per group were boosted three weeks after the primary immunization and were harvested two weeks later.

Immune Responses: Spleen cells were used in a CTL assay, an ICC assay for CD8+ and CD4+ T-cell responses, and for IFN-gamma. Sera were used to measure Mtb8.4 specific antibodies. These methods are the same as those used in Examples 1-7 above.

### Results

Microsphere Characterization: The results of the microsphere *in vitro* physico-chemical analysis are shown in Table 2. The Mtb8.4 protein core-loadings for the protein-microspheres (a), protein-MPL co-encapsulated microspheres (d), and protein-529 co-encapsulated microspheres (e) were similar and ranged from 0.62%-0.87%. The MPL core-loadings for the MPL-microspheres (b) and the protein-MPL co-encapsulated microspheres (d) were 0.64% and 0.70%, respectively, while the loadings of the 529-microspheres (c) and the protein-529 co-encapsulated microspheres (e) were 0.71% and 0.80%, respectively. All microspheres of this set were of similar size, with diameters of approximately 1 µm. Table 2 also shows that the amount of protein released at the 2-hour time point was 3% for the protein microspheres and 17% and 21 % for the protein-MPL and protein-529 co-encapsulated microspheres.

**Table 2: Characterization of Mtb8.4, MPL, and RC529 Formulations**

| | **Description** | **Lot** | **Mtb8.4** | **Adjuvant** | | **Adj/Mtb8.4** | **Diameter** | **Mtb8.4 Released** |
|---|---|---|---|---|---|---|---|---|
| | | | **CL** | | **CL** | **Ratio** | **(µm)** | **at 2-hrs** |
| (a) | **(Mtb8.4)-MS** | MJ087 | **0.82%** | **--** | **--** | **--** | **0.8** | **3%** |
| (b) | **(MPL)-MS** | MJ092 | **--** | **MPL** | **0.64%** | **--** | **0.8** | **--** |
| (c) | **(529)-MS** | MJ091 | **--** | **529** | **0.71%** | **--** | **0.8** | **--** |
| (d) | **(Mtb8.4+MPL)-MS** | MJ102 | **0.87%** | **MPL** | **0.70%** | **0.80** | **0.9** | **17%** |
| (e) | **(Mtb8.4+529)-MS** | MJ090 | **0.62%** | **529** | **0.80%** | **1.29** | **0.9** | **21%** |

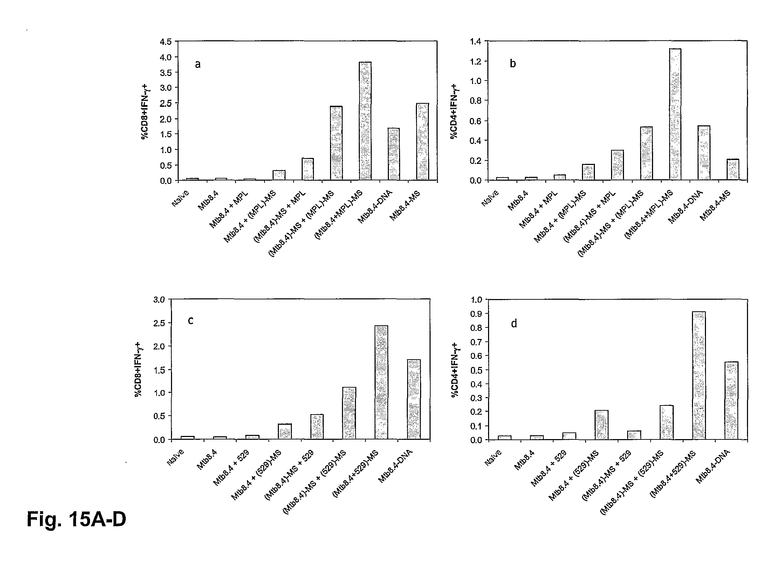

ICCS Results: The CD8+ and CD4+ T-cell response results from the ICCS assays performed on spleen cells harvested after only one immunization are shown in Figures 15A-D. Figures 15A and 15B represent the Mtb8.4 specific CD8 and CD4 T-cell responses for the MPL and control groups while Figures 15C-D represent the results for the 529 and control groups. All four figures show that the strongest CD8 and strongest CD4 T-cell responses were generated by the protein-adjuvant co-encapsulated microspheres with either MPL or 529. For both CD8 and CD4 T-cell responses, the protein-MPL co-encapsulated microspheres elicited stronger responses than did the protein-529 co-encapsulated microspheres. Essentially no CD8 or CD4 T-cell responses were elicited by Mtb8.4 protein alone, protein plus MPL® adjuvant, or naive mice. Figures 15A-D also show that adjuvant-microspheres admixed with either protein alone (groups F and K) or with protein-microspheres (groups H and M) enhances both CD8 and CD4 T-cell responses above those generated by adjuvant formulated in the -AF lipid formulation (groups E, J, G, and L). That is, adjuvant-microspheres is an effective adjuvant formulation for both naked protein and for protein-microspheres using both MPL and 529.

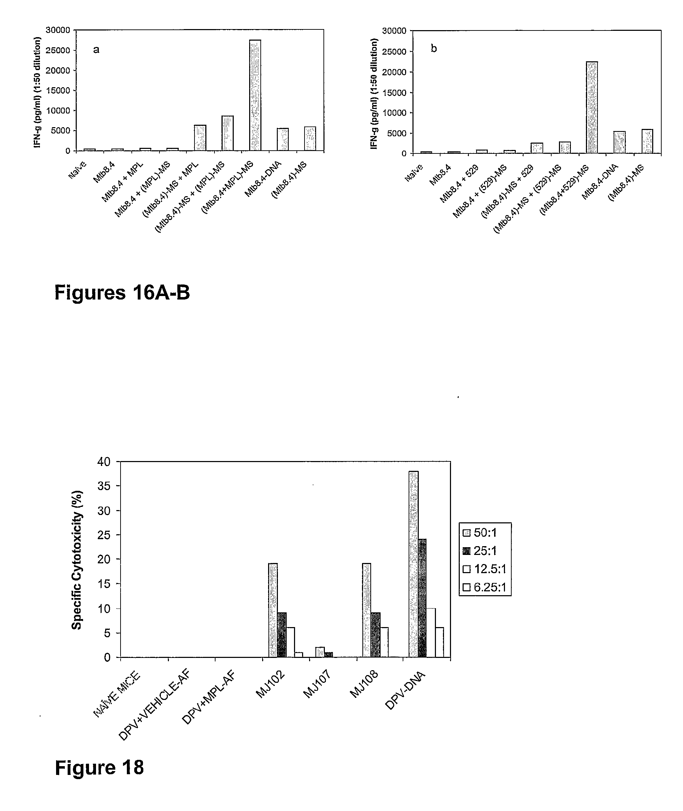

IFN-y Results: The IFN-γ release from spleen cells harvested two-weeks after the primary immunization are shown in Figure 16A (MPL and control groups) and 16B (529 and control groups). Figures 16A-B show that, as with the CD8 and CD4 T-cell responses, the strongest IFN-γ responses are elicited by the protein-MPL and protein-529 co-encapsulated microspheres, with the protein-MPL co-encapsulated microsphere formulation again eliciting the stronger of the two responses. In this assay, the groups that received protein-microspheres, whether admixed with adjuvant or adjuvant-microspheres or not (groups D, G, H, L, and M), all elicited comparable responses that were clearly greater than those elicited by protein alone and protein plus adjuvant (groups B, E, F, J, and K) but substantively lower than by those elicited by the co-encapsulated groups (I and N).

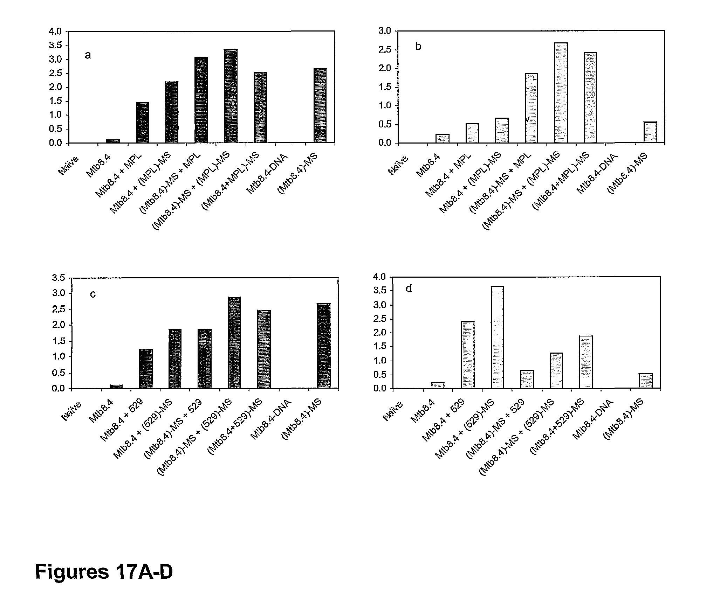

IgG1 and IgG2b Antibody Responses: IgG1 and IgG2b antibody responses measured from sera obtained two-weeks after the secondary immunization are shown in Figures 17A-B for the control groups and those receiving MPL, and in Figures 17C-D for the control groups and those receiving 529. Responses shown are the OD measured at the 16000:1 dilution for IgG1 and 4000:1 dilution for IgG2b. Figures 17A and 17C shown that the strongest IgG1 responses were obtained for the groups receiving protein encapsulated in microspheres (groups G, H, I, L, M, and N). DNA and protein alone immunizations elicited little to no IgG1 and protein admixed with either MPL-AF and 529-AF elicited the weakest IgG1 responses. Figure 17B shows that the strongest IgG2b responses were obtained from protein-microspheres combined with adjuvant (groups G, H, and I). These responses were much greater than those elicited by DNA, protein alone, protein admixed with MPL-AF, and protein mixed with MPL-microspheres. Interestingly, in every comparison in Figure 17A-D of adjuvant formulated in lipids versus adjuvant formulated in microspheres (e.g., E vs. F, G vs. H, J vs. K, and L vs. M for IgG1 and for IgG2b) the adjuvant-microsphere groups elicited higher levels of antibodies. This again shows that adjuvant- microspheres are an effective formulation of adjuvant and possibly better than the -AF lipid formulation.

These data show that protein-adjuvant co-encapsulation can be the most effective vaccine formulation for eliciting comprehensive immune responses, including CD8 T-cell responses, CTL, CD4 T-cell responses, IFN-γ, and IgG1 and IgG2b antibody responses. Co-encapsulation can be effective with both MPL, as shown in the previous examples, as well as with RC529, a synthetic AGP. Based on these results, it would be expected that other synthetic adjuvants would also be effective in a microsphere co-encapsulated formulation. These data suggest that, at least for Mtb8.4, MPL is a slightly better adjuvant than 529 for co-encapsulating in microspheres.

These data also clearly demonstrate that adjuvant-microspheres are an effective formulation of adjuvants. This was shown for both MPL and 529. In comparison with - AF Liposomal adjuvant formulations, adjuvant-microspheres performed as well if not better at enhancing immune responses. Development and optimization of the adjuvant-microsphere formulation is promising for creating the most effective vaccine adjuvant formulation.

### Example 9: Alternate and optimal methods of Mt8.4 and MPL co-encapsulation

This example demonstrates that alternate methods of protein and adjuvant co-encapsulation can still produce microspheres capable of eliciting strong, comprehensive immune responses. Specifically, microspheres were made using a double-emulsion technique, and compared to co-encapsulated microsphere that were prepared using single-emulsion techniques. In addition, the example demonstrates an alternative, and more manufacturing friendly way, to apply HIP that produces effective microspheres. In this method, an aqueous protein solution is used as the inner aqueous phase in the double emulsion process with the HIP agent in the solvent. Presumably the protein is extracted into the solvent during the primary emulsion step.

Alternate methods for preparing protein-adjuvant co-encapsulated microspheres were examined. These methods were evaluated in terms of their ability to yield microspheres with reasonable encapsulation efficiencies (i.e., the percent of input reagent in the final microsphere product) and to stimulate comprehensive immune responses in animals. The protein examined was Mtb8.4 ("DPV"), a *Mycobacterium tuberculosis* antigen and the adjuvant was MPL® adjuvant.

Microsphere Preparation: Mtb8.4-MPL co-encapsulated microspheres were prepared using three different techniques but with several features in common. That is, all three formulations used 3 mg of Mtb8.4 protein, 3 mg of MPL® adjuvant, 300 mg of RG502H polymer, 400 ml of 5% PVA as the process media, and utilized a Silvers on mixer to prepare the microspheres with a mixing speed of 9000 rpm. The first microsphere formulation, lot #MJ102, was prepared as described in examples 1, 6 and 7. In brief, Mtb8.4 protein was extracted from an aqueous solution into a docusate sodium-dichloromethane (DCM) solution via hydrophobic ion pairing. The aqueous solution was then discarded. Polymer and MPL® adjuvant were added to the DCM and microspheres were prepared. The second formulation, lot #MJ107, utilized a modified solvent system in order to solubilize the protein in the organic phase without using HIP and/or docusate sodium. Mtb8.4 by itself is insoluble in DCM. That is, Mtb8.4 was lyophilized from an aqueous solution at acidic pH and dissolved in dimethyl sulfoxide (DMSO) to which an equal volume of DCM was added. Polymer and MPL® adjuvant were then dissolved in the solvent phase and microspheres were prepared. The third formulation, lot #MJ108, utilized a variation on the double emulsion technique. The internal aqueous phase was a 1.0 ml aqueous solution of Mtb8.4 protein at acidic pH, as with the extraction for MJ102. The organic phase was DCM containing approximately 8 mg of docusate sodium, as with the extraction for MJ102, and the polymer and MPL® adjuvant. The primary emulsion was formed by vigorously mixing the protein solution in the solvent phase using a vortex. Presumably the protein was extracted into the organic phase via hydrophobic ion pairing, as with MJ102. However this technique is simpler in that the aqueous phase does not need to be separated and discarded, a cumbersome aspect of the MJ102 process. Microspheres were then prepared using the primary emulsion as described above.

Microsphere Characterization: The protein and adjuvant contents of the microspheres, were determined by HPLC or amino acid analysis and the Bartlett inorganic phosphorous assay. Size distributions were measured using a Horiba LA920 and through visual estimations from scanning electron micrographs. Release kinetics were measured by dispersing 10-20 mg of formulation in 100 mM Tris buffer, sampling the supernatant over time, and quantifying the protein concentration by reverse phase HPLC.

Immunizations: Groups of seven (7) C57B1/6 were immunized with 5 µg of encapsulated Mtb8.4 protein per mouse. The target dose of MPL® adjuvant was 5 µg per mouse. The actual dosings were 4.1, 4.3, and 6.4 µg per mouse for MJ102, MJ107, and MJ108, respectively (Table 3). Control groups of mice include naive, Mtb8.4 protein plus MPL-AF, protein plus the -AF vehicle (lipids in water), and Mtb8.4-DNA. All protein formulations were administered by the intradermal route while DNA was administered intramuscularly. Three or four mice from each group were harvested two weeks after priming, with their spleens and sera collected and pooled for evaluation. The remaining three or four mice per group were boosted three weeks after priming and harvested two weeks later.

Immune Responses: Spleen cells were used in a CTL assay, an ICCS assay for CD8+ and CD4+ T-cells responses, and for IFN-gamma. Sera were used to measure Mtb8.4 specific antibodies. These methods are the same as those used in Examples 1-7 above.

### Results

Microsphere Characterization: The protein and MPL® contents ("core-loading," abbreviated as "CL") and encapsulation efficiencies are listed in Table 3. The encapsulation efficiencies for the protein were similar and reasonable, ranging from 64-76% for MJ102, MJ107, and MJ108. The MPL® adjuvant encapsulation efficiencies were also similar and reasonable in value, ranging from 62-79%. The adjuvant-to-antigen ratios were also all close to unity, ranging from 0.81 to 1.28.

**Table 3: Mtb8.4 and MPL® Adjuvant Core-loadings and Encapsulation Efficiencies**

| **Lot** | **Mtb8.4 Protein** | | **MPL® Adjuvant** | | **Adj/Ant** | **Immunizations (ug)** | |
|---|---|---|---|---|---|---|---|
| | **CL** | ε**-_{CL}** | **CL** | ε**-_{CL}** | **Ratio** | **Mtb8.4** | **MPL** |
| **MJ102** | **0.87%** | **76%** | **0.71%** | **69%** | 0.81 | 5.0 | 4.1 |
| **MJ107** | **0.73%** | **74%** | **0.63%** | **62%** | 0.86 | 5.0 | 4.3 |
| **MJ108** | **0.62%** | **64%** | **0.80%** | **79%** | 1.28 | 5.0 | 6.4 |

The median particle diameters and the initial protein release kinetic data for these three formulations are shown in Table 4. The median diameters for these formulations are between 1 and 2.6 µg, well within the size range desired for optimal phagocytosis of microspheres (i.e., ∼1-10 µm). Table 4 also shows that the two formulations containing AOT, MJ102 and MJ108, exhibit significantly lower initial release kinetics than MJ107. That is, 78% of the encapsulated protein for MJ107 is released within 2 hours while only approximately 20% of the protein is released from MJ102 and MJ107. This may be due to (i) hydrophobic ion pairing of the protein with docusate sodium, (ii) affects of AOT on the formulation, and/or (iii) the use of a more hydrophilic solvent (DMSO:DCM 1:1) for MJ107. Excessive initial release of the protein may reduce the efficacy of the microsphere by not allowing sufficient time for phagocytosis of the microspheres prior to release.

**Table 4: Microsphere Sizes and Initial Protein Release Kinetics**

| **Lot** | **Median** | **% Protein cReleased** | | |
|---|---|---|---|---|
| | **Diam (um)** | **2-hrs** | **24-hrs** | **48-hrs** |
| **MJ102** | **1.0** | **17%** | **15%** | **15%** |
| **MJ107** | **2.4** | **78%** | **78%** | **77%** |
| **MJ108** | **2.6** | **23%** | **22%** | **22%** |

CTL Responses: CTL responses measured two weeks after a single immunization are shown in Figure 18. Mice receiving Mtb8.4 protein plus MPL® adjuvant exhibited no significant specific CTL responses. Likewise, naive mice and mice receiving protein plus the -AF vehicle (lipids) failed to elicit specific CTL responses. However, the Mtb8.4 protein-MPL co-encapsulated formulation that previously was proven effective (See Examples 1, 6, 7, and 8), MJ102, again successfully elicited CTLs. Likewise, co-encapsulated microspheres prepared using the double emulsion technique, MJ108, elicited a CTL response similar in strength. MJ107, the co-encapsulated formulation with nearly 80% release at 2 hours, did not elicit a strong CTL response at this time-point in this experiment.

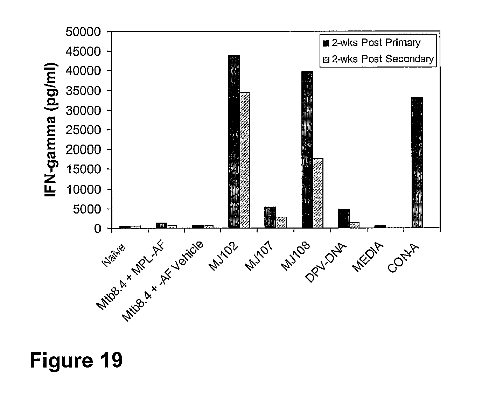

IFN-gamma Responses: IFN-gamma release from spleen cell cultures measured from mice receiving either a primary immunization or a primary and a secondary immunizations are shown in Figure 19. Figure 19 shows that essentially no IFN-gamma was elicited in mice receiving protein plus MPL® adjuvant, protein plus -AF vehicle, or naive mice. Mice receiving DNA elicited IFN-gamma responses barely above background. In contrast, MJ102 and MJ108 microspheres both elicited strong and comparable IFN-gamma responses in mice receiving either one or two immunizations. As with CTL responses, MJ107 microspheres elicited lower IFN-gamma than the other two microsphere formulations having lower initial release kinetics but levels above background and comparable to those in the DNA group.

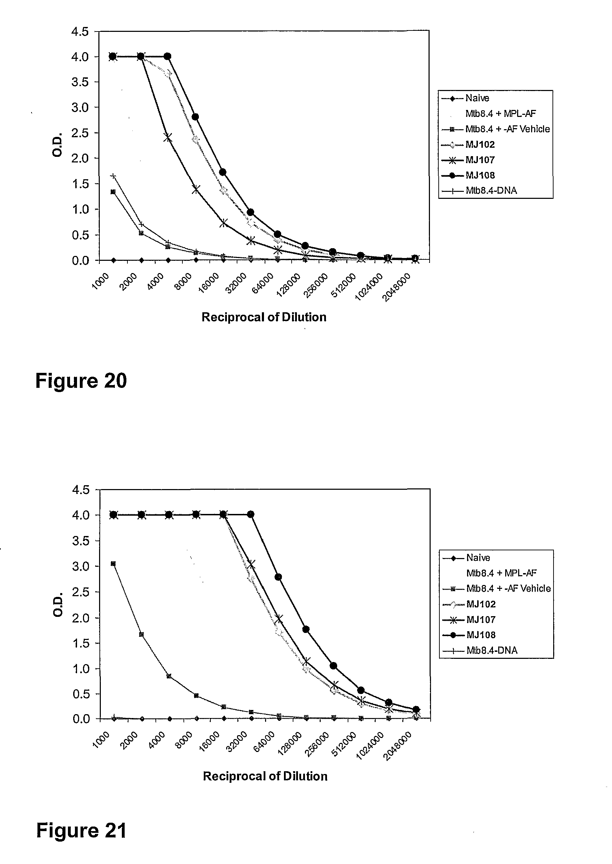

IgG1 and IgG2b Antibody Responses: Figures 20 and 21 show the Mtb8.4 specific IgG2b and IgG1 antibody responses elicited in mice, respectively. Figure 20 shows that the three microsphere formulations elicited the strongest IgG2b responses. Protein plus MPL® adjuvant and Mtb8.4-DNA elicited lower levels of IgG2b than did the microspheres. As with the CTL and IFN-gamma responses, MJ102 and MJ108 elicited stronger responses than MJ107. Similarly, Figure 21 shows that the three microsphere formulations elicited the strongest IgG1 specific antibody responses. Protein plus MPL® adjuvant and Mtb8.4-DNA elicited lower levels of IgG1 than did the microspheres. In contrast to the other immune response measurements, the IgG1 responses for the three microsphere formulations were all comparable.

These data show that there are multiple manifestations of co-encapsulated microspheres (HIP extractions, admixing protein and adjuvant in a solvent, double emulsion) that exhibit good microsphere properties (size, encapsulation efficiencies, release kinetics, etc.) and elicit strong immune responses in mice, including CD8 T-cells, CTL, CD4 T-cells, IFN-gamma, and IgG2b and IgG1 antibodies. These data also show that there are multiple manifestations of hydrophobic ion pairing (e.g., extractions, double emulsions) that also yield good microsphere properties (size, encapsulation efficiencies, release kinetics, etc.) and elicit strong immune responses in mice, including CD8 T-cells, CTL, CD4 T-cells, IFN-gamma, and IgG2b and IgG1 antibodies. In addition, the lesser performance of MJ107 relative to MJ102 and 1008 shows that the formulation process mater and the properties of the resulting product are not always predictable *a priori*. Importantly, MJ107 still performed better than protein plus adjuvant in all assays examined.

Both of the alternate methods examined in this example (MJ107: co-solubilizing the protein and adjuvant in organic solvent, and MJ108: double emulsion as a pseudo-extraction that is more manufacturing friendly than that used for MJ102) are techniques that are simpler to use in the lab and, presumably, simpler to manufacture.

### Example 10: 72f Protein Microspheres Elicit Strong Immune Responses

This example demonstrates several points related to the optimization of delivery of a preferred tuberculosis antigen, 72f. 72f is a fusion of three polypeptides: RA12, TbH9, and RA35. The example shows that: (1) recombinant 72f (r72f) protein encapsulated in microspheres can elicit strong CD4+ and CD8+ T-cell responses, CTL and antibody responses; (2) co-encapsulation of r72f and adjuvant (MPL) elicits stronger immune responses than r72f-microsperes without adjuvant; (3) double emulsion and single emulsion formulations both elicit the strongest immune responses; and (4) CD4+ T-cell responses (Elispot) elicited by the best microspheres are dramatically stronger than those elicited by protein plus AS-2 (MPL and saponin), protein plus MPL, and protein alone.

A series of initial PLG microsphere formulations of the *M. tuberculosis* fusion protein 72f were prepared and evaluated *in vivo.* The formulation methods and materials were chosen in order to vary the interactions of the protein with itself, polymer, the solvents etc. during microsphere formation and in the final product. The differences are designed to produce different microsphere properties in terms of *in vitro* characterization and, moreover, *in vivo* evaluation of their ability to generate comprehensive antigen specific immune responses. Two of these formulations had protein and MPL® adjuvant co-encapsulated. Immune responses were measured for each of the three constituents of the 72f fusion protein (see Immune Responses below).

Microsphere Preparation: 72f protein microspheres were prepared using single and double emulsion techniques (Table 5). All formulations described in Table 1 had 300 mg of a PLG polymer, 3 mg of 72f protein, 10 ml of organic solvent (Table 5), and were emulsified using a Silverson mixer at 9000 rpm to produce microspheres. Difference in the formulations included single emulsion (SE) versus double emulsion (DE) processes, carboxylic acid versus ester end group chemistry on the polymer, the polymer end group frequency (i.e., molecular weight: ∼10 kDa versus ∼40 kDa), DCM versus DCM:DMSO (35:65), addition of MPL® adjuvant, process media volume (280 versus 400 ml), and stabilizer (CMC 1.4% versus PVA 5%). Note that AM123 was prepared in the same manner as AM117 with the exception of the co-encapsulation of MPL® adjuvant in AM123. Likewise, AM124 was prepared in the same manner as AM120 with the exception of the co-encapsulation of MPL® adjuvant in AM124.

**Table 5: Parametric Variations in the Preparation of the 72f Protein Microspheres**

| **Lot** | **Method** | **Internal H2O** | **PLG** | **Organic Solvent** | **Additive** | **Process Media** | |
|---|---|---|---|---|---|---|---|
| | | **Volume (ml)** | **Type** | | | **Vol (ml)** | **Stabilizer** |
| **AM116** | SE | -- | 502H | DCM:DMSO (35:65) | -- | 280 | CMC (1.4%) |
| **AM117** | SE | -- | 502H | DCM:DMSO (35:65) | -- | 400 | PVA (5%) |
| **AM118** | SE | -- | 503 | DCM:DMSO (35:65) | -- | 400 | PVA (5%) |
| **AM119** | DE | 3.3 | 502H | DCM | -- | 400 | PVA (5%) |
| **AM120** | DE | 3.3 | 503 | DCM | -- | 400 | PVA (5%) |
| **AM123** | SE | -- | 502H | DCM:DMSO (35:65) | MPL | 400 | PVA (5%) |
| **AM124** | DE | 3.3 | 503 | DCM | MPL | 400 | PVA (5%) |

Microsphere Characterization: The protein and adjuvant contents of the microspheres were determined by amino acid analysis and the Bartlett inorganic phosphorous assay. Size distributions were measured using a Horiba LA920 and through visual estimations from scanning electron micrographs.

Immunizations: Groups of three C57BL/6mice were immunized twice, three weeks apart with 10 µg of encapsulated 72f protein per mouse at the base of the tail (i.e., ID). Spleens and sera were harvested from the mice two weeks post second immunization for evaluation of immune response generation. The target dose of MPL® adjuvant was 10 µg per mouse. The actual dosings of AM123 and AM124, the two microsphere formulations with protein and MPL® adjuvant co-encapsulated, were 5 and 12 µg, respectively. Control groups included naive mice, protein alone (10 µg), protein plus AS-2 (MPL® and saponin) in an oil-in-water emulsion, and protein plus MPL®-SE adjuvant.

Immune Responses: Spleen cells were used in Elispot and CTL assays. Cells were stimulated with TbH9 protein, RA12 protein, RA35 protein, and a CD8 10 amino acid Db epitope to RA12 for the Elispot assay. Cells were stimulated with EL4 cells transduced with Ra12 protein for the CTL assay. Antigen specific ELISAs were used to measure antibodies to TbH9 protein, RA12 protein, and RA35 protein in sera.

### Results

Microsphere Characterization: The protein and MPL® contents ("core-loading," or "CL") are listed in Table 6. The protein core-loadings were similar, ranging from 0.63% to 1.35%. The MPL® adjuvant core-loadings for AM123 and AM124 were 0.68% and 0.93%, resulting in the administration of approximately twice as much adjuvant for AM124 than for AM123, given a fixed protein dose (i.e., 10 µg). Table 6 shows that the sizes of the microspheres were also similar at approximately 1 µm in diameter.

**Table 6: 72f and MPL® Adjuvant Core-loadings and Encapsulation Efficiencies**

| **Lot** | **Est Diam (um)** | **Core-Loadings** | | **Adj/Ant Ratio** | **immunizations (ug)** | |
|---|---|---|---|---|---|---|
| | | **r72f** | **MPL®** | | **72f** | **MPL** |
| **AM116** | **0.8** | **1.26%** | **--** | -- | 10.0 | -- |
| **AM117** | **1.0** | **1.14%** | **--** | -- | 10.0 | -- |
| **AM118** | **1.5** | **0.63%** | **--** | -- | 10.0 | -- |
| **AM119** | **1.5** | **1.23%** | **--** | -- | 10.0 | -- |
| **AM120** | **1.0** | **1.17%** | **--** | -- | 10.0 | -- |
| **AM123** | **1.5** | **1.35%** | **0.68%** | 0.50 | 10.0 | 5.0 |
| **AM124** | **1.0** | **0.79%** | **0.93%** | 1.18 | 10.0 | 11.8 |

Elispot Assay: The results of the Elispot assay are based on triplicates obtained from the pooled spleen cells of three mice. The numbers indicated represent the number of IFN-γ Elispots per well. Negative controls for the assay where cells from each mouse in each group that were stimulated with medium alone. Each of the medium stimulated wells had few (<15) positive spots. In contrast, cells stimulated with TbH9 recombinant protein (10 µg/ml) showed positive but varied responses between groups. That is, naive mice ("medium" row), and mice receiving protein alone, failed to elicit a TbH9 specific positive response. Protein formulated with AS-2 as well as protein plus MPL®-SE adjuvant both elicited positive TbH9 specific responses, with an average of 52 and 154 spots per well, respectively. All protein-microsphere groups also elicited positive TbH9 specific responses with average spots per well for AM116, AM117, AM118 and AM119 being 43, 41, 77, and 69, respectively. However, protein microsphere formulations AM120 (no MPL) and AM123 and AM124 (MPL co-encapsulated) elicited such strong TbH9 responses that the there was a confluence of spots across the wells. Comparison of AM117 (no MPL) and AM123 (MPL co-encapsulation) clearly demonstrates the dramatic enhancement in immune responses generated due to MPL co-encapsulation. Whereas AM120 (no MPL) responses are already maximal, no difference can be seen with AM124 (MPL co-encapsulation). No Ra35 nor RA12 specific responses were observed, relative to naive mice. The strongest responses to the Ra12 CD8+ T cell epitope (peptide #86-95) were again observed with microspheres. For this read out, the responses were fairly uniform for six of the eight formulations (AM 116, AM117 AM119, AM120, and AM123) while AM118 and AM124 exhibited slightly lower responses. In short, protein microspheres generated the strongest CD4+ T cell responses to TbH9 as well as CD8+ T cell responses to the Ra12 epitope. These responses were far stronger than those of protein alone and stronger than those elicited by MPL and AS-2 adjuvant systems.

CTL Responses: Secondary CTL responses were measured against Ra12 two weeks after the second immunizations. Mice receiving 72f protein plus MPL® adjuvant exhibited no significant specific CTL responses. No specific lysis was observed for naive mice and mice receiving protein alone. Protein plus AS-2 generated CTL responses in 3/3 mice, with moderate to high levels of background. All of the protein-microsphere formulations elicited CTL responses, with the sole exception of AM118. The strongest, most consistent CTL responses were to AM123, AM116, AM117 and AM119. Comparison of AM117 (no MPL) with AM123 (MPL co-encapsulation) again demonstrates the substantive enhancement of immune responses generation by co-encapsulating MPL. In short, protein microspheres clearly generated stronger CTL responses than protein alone and protein plus MPL® adjuvant.

Ra12 specific antibody responses in individual mice were measured two weeks after the second administration of 10 µg formulated 72f protein. IgG1 responses were all strong and fairly similar for all of the mice immunized with a protein formulation. IgG2a responses were observed in select groups and were lower than IgG1. Protein plus MPL generated IgG2a in 3/3 mice and protein plus AS-2 generated fairly robust IgG2a in 3/3 mice. One microsphere formulation not having MPL co-encapsulated generated IgG2a, that being AM120, with 2/3 responders. The two microsphere formulations having protein and MPL® adjuvant co-encapsulated, AM123 and AM124, generated the strongest responses out of the microsphere groups with 3/3 responders each. IgG1 responses against both TbH9 and Ra35 were similar for the microsphere formulations and for protein plus AS-2, while responses to protein alone and protein plus MPL (for Ra35) were lower. Minimal IgG2a was elicited against TbH9 and Ra35.

These data clearly demonstrate the ability of protein-microsphere formulations to enhance immune responses, both CD8+ T cell, CD4+ T cell, and antibody, over those elicited by protein alone for the *M. tuberculosis* fusion protein and product candidate 72f. These enhancements can be dramatic. These data also demonstrate how different microsphere formulations, including microspheres made by single and double emulsion processes, can produce different immune responses *in vivo.* Protein and MPL® adjuvant co-encapsulated in microspheres can be a highly effective vaccine delivery system for obtaining comprehensive immune responses.

### Example 11: rICD (Her-2/neu) Protein Microspheres in Tumor Protection Assay

This example demonstrates that recombinant her-2/neu intracellular domain (rICD) protein microspheres can produce effective tumor protection. Several (n=4) ICD-protein microsphere formulations were prepared. All were of similar size, produced using the same lot of protein, and contained a version of PLG polymer as the microsphere matrix. However, different polymers, additives and techniques were utilized to produce microspheres with different properties, as measured either *in vitro* or *in vivo.* In particular, these approaches were designed to vary the interactions of the protein with itself, with the polymer, with an additive, and to vary the interactions of the solvents, polymer, stabilizers and water with each other, both during the manufacturing process, as well as in the final microsphere product. None of these formulation contained adjuvant. These four formulations were evaluated in a tumor protection model.

Microsphere Preparation: Four different ICD protein microsphere formulations were prepared, characterized, and evaluated in a tumor protection model. These formulations had several features in common. All three formulations used 2.5 mg of ICD protein, 300 mg of PLG polymer, 5% PVA as the process media, and a Silverson mixer to prepare the microspheres. The formulation parametric variations for these microspheres are summarize in Table 7. These included single emulsion and double emulsion techniques, polymer end-group type (carboxylic acid "H" vs. ester end groups), polymer end group frequency (i.e., polymer molecular weight: ∼10k and ∼40K), the solvent system used (DCM and DMC:DMSO (1:3.3)), the process media volume, the mixing speed used (7000 and 9000 rpm), and the addition of an additive (ethyl stearate).

**Table 7: Parametric variations in the preparation of the ICD protein microspheres**

| **Lot** | **Method** | **Internal H2O** | **PLG** | **Organic Solvent** | | **Additive** | **Process** | **Mixing Speed** |
|---|---|---|---|---|---|---|---|---|
| | **ε_{-CL}** | **Volume (ml)** | **Type** | | **Vol (ml)** | | **Media (ml)** | **(rpm)** |
| **AM049** | SE | - | 502H | DCM:DMSO (1:3) | 13 | - | 400 | 9000 |
| **AM050** | SE | - | 503 | DCM:DMSO (1:3) | 13 | - | 400 | 9000 |
| **AM051** | DE | 2.0 | 502H | DCM | 10 | - | 400 | 9000 |
| **AM052** | DE | 2.0 | 502H | DCM | 10 | Ethyl Stearate (60 mg) | 20 | 7000 |

Microsphere Characterization: The protein contents of the microspheres were determined by amino acid analysis. Size distributions were measured using a Horiba LA920 and through visual estimations from scanning electron micrographs.

Immunizations: Groups of eight (8) C57B1/6 mice were immunized twice, three weeks apart, with 25 µg of encapsulated ICD protein per mouse. Control groups received 25 µg of ICD formulated in Montanide, an adjuvant previously determined to perform well with this system, ICD-DNA, and naïve mice. Protein formulations were administered by the intradermal route while DNA was administered intramuscularly.

Tumor Challenge: Two weeks after receiving the second immunizations mice were challenged with EL4-Her-2/neu tumor cells. Tumor sizes were measured periodically over six weeks.

### Results

Microsphere Characterization: The protein and contents ("core-loading," abbreviated CL) and microsphere diameters for AM049, AM050, AM051, and AM051 ICD-microspheres are listed in Table 8. Table 8 shows that these microsphere formulations had similar core-loadings, ranging from 0.96% to 1.24%, and were of similar sizes.

**Table 8: ICD Formulation Characterization**

| **Lot** | **ICD CL** | **Microsphere Diam (um)** |
|---|---|---|
| **AM049** | **1.24%** | **2.0** |
| **AM050** | **1.14%** | **1.0** |
| **AM051** | **0.96%** | **1.0** |
| **AM052** | **1.13%** | **0.8** |

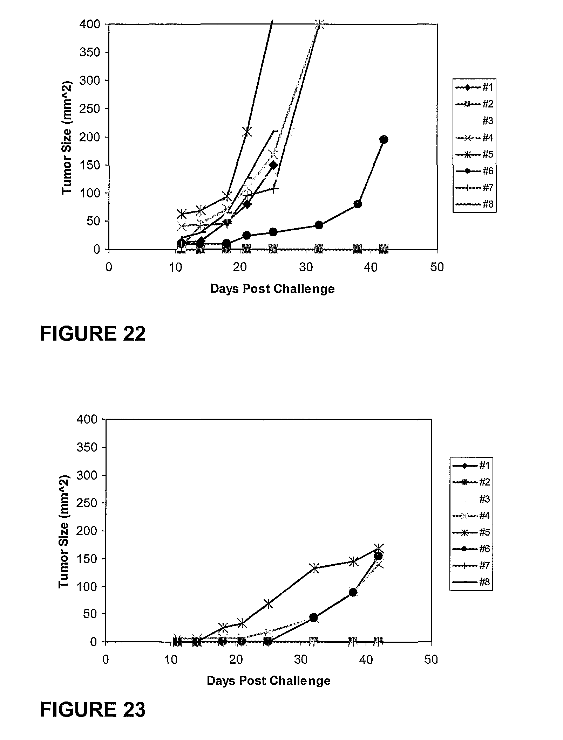

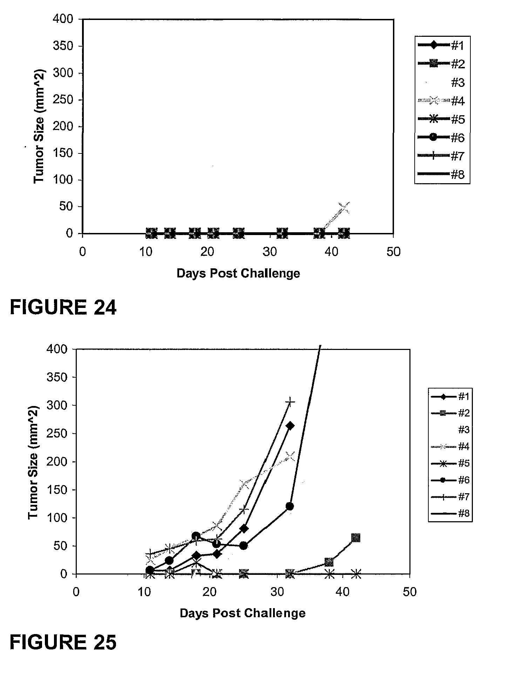

Tumor Protection: Figures 23-29 show the results of the tumor protection experiment. Figure 22 shows the tumor progression in naive mice. Six of eight mice had tumor growth shortly after tumor implantation with seven of eight mice exhibiting significant tumor growth by the end of the experiment. Figures 23 and 24 show tumor growth in the positive control groups that received ICD protein in Montanide and ICD-DNA, respectively. Figure 23 shows that ICD formulated in Montanide adjuvant, an adjuvant that has previously been found to be effective in this tumor model, exhibited enhanced protection relative to naive mice. By week 6, 3/8 mice in the Montanide group had significant tumor growth. ICD-DNA, the most consistent positive control in previous experiments, resulted in significant tumor protection (Figure 24). No mice receiving the DNA immunizations had measurable tumor growth until nearly the end of the experiment, at which time one mouse was found to be growing tumor.

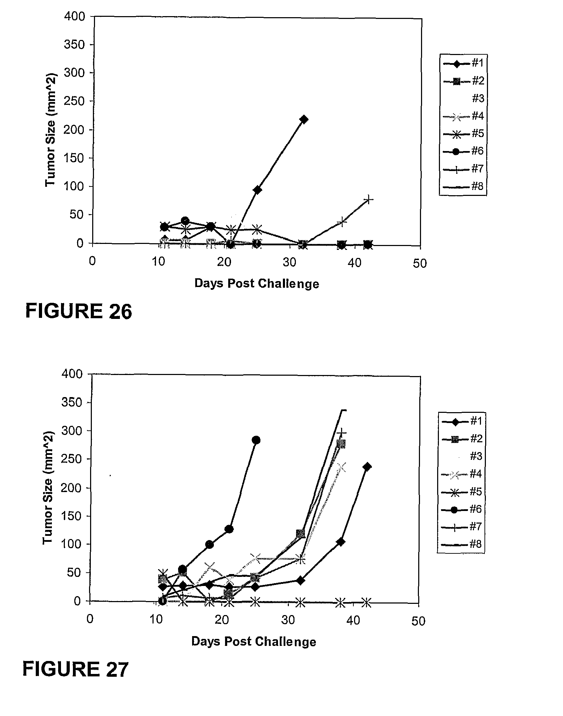

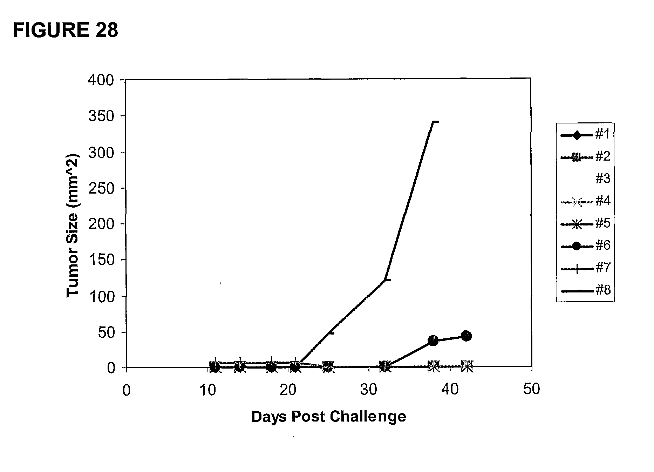

Figures 25-28 show the results of the groups of mice (n=8) which were immunized twice with 25 µg of ICD protein encapsulated in formulation number AM049, AM050, AM051, or AM052, respectively. In comparing these data to the control groups, ICD-protein microsphere lot AM049 (Figure 25) appears to have produced little protection relative to the naive group (Figure 22) while AM051 (Figure 27) appears to have delayed the onset of tumor growth somewhat. In contrast, ICD protein microsphere lot AM050 produced significant protection (Figure 26). By week 6, only 2/8 mice had significant tumor growth, a result comparable to if not slightly better than the Montanide adjuvant group. Moreover, ICD protein microsphere lot AM052 (Figure 28) appears to have produced the strongest and most consistent protection results for any of the protein formulations. By week 6, only 1/8 mice had significant tumor growth with a second mouse having just begun to show tumor growth. Moreover, the background tumor sizes measured for the first four weeks was extremely low, similar to that produced by the DNA immunizations (Figure 25).

These data clearly demonstrate the efficacy that protein microspheres can have as a vaccine delivery system, with the best tumor protection data for protein formulations being generated by microspheres. These data also illustrate how different microsphere formulations can produce different immune responses.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended claims.

### CLAUSES

1. A pharmaceutical composition comprising an adjuvant encapsulated in biodegradable polymeric microspheres and a pharmaceutically acceptable carrier.
2. The pharmaceutical composition of claim 1 further composing a protein co-encapsulated with the adjuvant in the microspheres.
3. The pharmaceutical composition of claim 1, wherein the adjuvant comprises MPL, AS-2, saponin, aluminum phosphate, calcium phosphate, aminoalkylghicosaminide phosphate, RC-529, RC-557, R544, isotucerasol, cell wall skeleton, and/or a CpG-containing oligonucleotide.
4. The pharmaceutical composition of claim 2, wherein the protein comprises an antigen associated with cancer, autoimmune disease or infectious disease.
5. The pharmaceutical composition of claim 4, wherein the infectious disease is tuberculosis.
6. The pharmaceutical composition of claim 5, wherein the antigen comprises Mtb8.4 or Mtb72f.
7. The pharmaceutical composition of claim 5, wherein the antigen comprises TbH9 (Mtb 39A), 38-1, Mtb41, Mtb40, Mtb32A, Mtb9.9A, Mtb9.8, Mtb16, Mtb59f, Mth88f, Mtb71f, Mtb46f or Mtb31f.
8. The pharmaceutical composition of claim 4, wherein the cancer is breast cancer.
9. The plarmaceutical composition of claim 8, wherein the antigen comprises ICD, ECD or ECD-PD of her-2/neu.
10. The pharmaceutical composition of claim 1, further comprising an adjuvant that is not encapsulated in the microspheres.
11. A method for encapsulating a protein into microspheres comprising.
   (a) solubilizing the protein in the presence of a hydrophobic ion pairing (HIP) agent and an organic solvent to produce an organic phase comprising the protein;
   (b) dissolving a polymer in the organic solvent or in the organic phase; and
   (c) preparing microapheres from a polymer solution, wherein the polymer solution comprises the organic phase, the protein, and the polymer.
12. The method of claim. 11, wherein the protein is extracted from an aqueous solution into the organic phase.
13. The method of claim 11, wherein the protein has a molecular weight of at least about 3 kDa.
14. The method of claim 11, wherein the protein has a molecular weight of at least about 8 kDa.
15. The method of claim 11, wherein the protein has a molecular weight of at least about 20 kDa.
16. The method of claim 11, wherein the protein has a molecular weight of at least about 50 kDa.
17. The method of claim 11, wherein the protein, has an amino acid sequence of at least about 20 amino acid residues.
18. The method of claim 11, wherein the protein has an amino acid sequence of at least about 60 amino acid residues.
19. The method of claim 11, wherein the protein has an amino acid sequence of at least about 80 amino acid residues.
20. The method of claim 11, wherein the protein has an amino acid sequence of at least about 100 amino acid residues.
21. The method of claim 11, wherein the solubilizing comprises combining the organic solvent with a dried HIP agent-piotein complex.
22. The method of claim 21, wherein the HIP agent-protein complex is dried by lyophilization or evaporation.
23. The method of claim 11, wherein the HIP agent is an anionic HIP agent
24. The method of claim 23, wherein the anionic HIP agent is docusate sodium.
25. The method of claim. 23, wherein the HIP agent is present in stoichiometric amount equal to or greater than the number of net positive charges on the protein.
26. The method of claim 11, wherein the HIP agent is a cationic HIP agent
27. The method of claim 26, wherein the cationic HIP agent is dimethyldioctadecylammonium bromide (DDAB18); 1,2-dioleoyloxy-3-(trimethylammonium)propane (DOTAP); or cetrimonium bromide (CTAB).
28. The method of claim 26, wherein the HIP agent is present in stoichiometric amounts equal to or greater than the number of net negative charges on the protein.
29. The method of claim 11, wherein the organic medium has a ratio of HIP agent to protein of up to about 70:1.
30. The method of claim 11, wherein the protein has a pI of at least about 7.0.
31. The method of claim 11, wherein the protein has a pI of at least about 7.5.
32. The method of claim 11, wherein the protein has a pI of at least about 8.0.
33. The method of claim 11, wherein the protein has a pI of up to about 6.0.
34. The method of claim 11, wherein the protein has a pI of up to about 6.5.
35. The method of claim 11, wherein the protein has a pI of up to about 7.0.
36. The method of claim 11, wherein the organic solvents comprises methylene chloride, dichloromethane, chloroform, ethylacetate, or dimethylsulfoxide.
37. The method of claim 11, wherein the aqueous solution has a total salt concentration of less than about 30 mM.
38. The method of claim 11, wherein the microspheres are prepared by a single oil-in-water emulsion.
39. The method of claim 11, wherein the microspheres are prepared by a double oil-in-water emulsion.
40. The method of claim 11, wherein the microspheres are prepared by spray drying or coacervation of the polymer solution.
41. The method of claim 11, wherein at least about 90% of the microspheres are about 1 to about 10 µm in diameter.
42. The method of claim 11, wherein the polymer comprises paly(lactide-co-glycolide) (PLG).
43. The method of claim 11, wherein the polymer comprises poly(lactide), poly(caprolactone),poly(hydroxybutyrate) and/or copolymers thereof.
44. The method of claim 11, wherein the polymer solution further comprises an adjuvant
45. The method of claim 11, wherein the polymer solution further comprises a cholesterol and/or a fatty acid ester.
46. The method of claim 45, wherein the fatty acid ester comprises ethyl myristate, ethyl caprate and/or ethyl stearate.
47. The method of claim 11, mother comprising the step of adding an adjuvant to said organic phase.
48. The method of claim 11, further composing the step of adding an adjuvant via an inner aqueous phase.
49. A pharmaceutical composition comprising a protein encapsulated in microspheres produced by the method of claim 11 and a pharmaceutically acceptable carrier.
50. A method for delivering an antigen to a subject comprising administering to the subject a composition of claim 2.
51. A method for eliciting an immune response to an antigen in a subject comprising administering to the subject a composition of claim 2.
52. The method of claim 51, wherein the immune response includes a cellular immune response and a humoral immune response.
53. A method for treating or preventing cancer in a subject comprising administering to the subject a therapeutically effective amount of a composition of claim 4.
54. A method for treating or preventing tuberculosis in a subject comprising administering to the subject a therapeutically effective amount of a composition of claim 4.

## Claims

1. A method for encapsulating a protein into microspheres comprising:
(a) solubilizing the protein in the presence of a hydrophobic ion pairing (HIP) agent and an organic solvent to produce an organic phase comprising the protein;
(b) dissolving a polymer in the organic solvent or in the organic phase; and (c)preparing microspheres from a polymer solution, wherein the polymer solution comprises the organic phase, the protein, and the polymer.

2. The method of claim 1 wherein the organic phase comprises an oil phase and the solubilising comprises partitioning the protein into the oil phase.

3. The method of claim 1 wherein the protein is extracted from an aqueous solution into the organic phase.

4. The method of claim 1 wherein the protein has a molecular weight of at least about 3kDa, preferably at least about 8kDa, more preferably at least 20kDa, most preferably at least 50kDa.

5. The method of claim 1, wherein the protein has an amino acid sequence of at least about 20 amino acid residues, preferably at least 60 amino acid residues more preferably at least 80 amino residues, most preferably at least 100 amino acid residues.

6. The method of claim 1 wherein the solubilizing comprises combining the organic solvent with a dried HIP agent-protein complex.

7. The method of claim 6 wherein the HIP agent-protein complex is dried by lyophilization or evaporation.

8. The method of claim 1 wherein the HIP agent is an anionic HIP agent or a cationic HIP agent.

9. The method of claim 8 wherein the anionic HIP agent is docusate sodium.

10. The method of claim 8 wherein the cationic HIP agent is dimethyldioctadecylammonium bromide (DDAB18); 1,2-dioleoyloxy-3-(trimethylammonium)propane (DOTAP); or cetrimonium bromide (CTAB).

11. The method of claim 8 wherein the HIP agent is present in stoichiometric amounts equal to or greater than the number of net negative or net positive charges on the protein.

12. The method of claim 1 wherein the organic medium has a ratio of HIP agent to protein of up to about 70:1.

13. The method of claim 1 wherein the protein has a pI of at least about 7.0, preferably at least about 7.5, most preferably at least about 8.0.

14. The method of claim 1 wherein the protein has a pI of up to about 6.0, preferably up to about 6.5, most preferably up to about 7.0.

15. The method of claim 1 wherein the organic solvent comprises methylene chloride, dichloromethane, chloroform, ethylacetate, or dimethylsulfoxide.

16. The method of claim 1 wherein the aqueous solution has a total salt concentration of less than about 30 mM.

17. The method of claim 1 wherein the microspheres are prepared by a single oil-in-water emulsion or a double oil-in-water emulsion.

18. The method of claim 1 wherein the microspheres are prepared by spray drying or coacervation of the polymer solution.

19. The method of claim 1 wherein at least about 90% of the microspheres are about 1 to about 10µ*m* in diameter.

20. The method of claim 1 wherein the polymer comprises poly-(lactide-co-glyc6lide) (PLG), poly(lactide), poly(caprolactone), poly(hydroxybutyrate) and/or copolymers thereof.

21. The method of claim 1 wherein the polymer solution further comprises an adjuvant.

22. The method of claim 1 wherein the polymer solution further comprises a cholesterol and/or fatty acid ester such as ethyl myristrate, ethyl caprate and/or ethyl stearate.

23. The method of claim 1, further comprising the step of adding an adjuvant to said organic phase or adding an adjuvant via an inner aqueous phase.
